# EUROPEAN PATENT APPLICATION

(11) **EP 4 574 143 A1**
(43) Date of publication of application: **25.06.2025**
(21) Application number: 23219442.3
(22) Date of filing: 21.12.2023
(51) Int. Cl.: A61K 31/14, A61K 31/4164, A61K 31/4425, A61K 45/06, A61P 33/06

(54) **INHIBITORS OF PLASMODIA REMODELING ENZYMES**

(71) Applicant: Universität Regensburg, 93053 Regensburg (DE); 2bind GmbH, 95053 Regensburg (DE)
(72) Inventor: The designation of the inventor has not yet been filed
(74) Representative: Engelhard, Markus

(57) **Abstract**

The invention is based on a compound for use in a method of diagnosis, prevention, and/or treatment of a Malaria infection in a subject, wherein the compound is an inhibitor of a Chromatin remodeling enzyme (CRE) of a Plasmodium or a related species of the Apicomplexa, Amoebozoa or Euglenozoa group, and wherein said CRE has a CRE ATPase domain. The invention also pertains to a pharmaceutical composition comprising the compound for use according to the first aspect of this invention, and a pharmaceutical acceptable carrier, stabilizer and/or excipient. The invention further relates to a pharmaceutical combination comprising: (i) the compound for use according to the first aspect of this invention, or the pharmaceutical composition according to the second aspect of this invention, and (ii) an additional anti-malarial compound. Further provided are kits comprising the compound for use according to the first aspect of this invention, the pharmaceutical composition according to the second aspect of this invention, or the pharmaceutical combination according to the third aspect of this invention, and technical instructions providing information on administration and/or dosage of the compound or the pharmaceutical composition.

## Description

### FIELD OF THE INVENTION

The invention is based on a compound for use in a method of diagnosis, prevention, and/or treatment of a Malaria infection in a subject, wherein the compound is an inhibitor of a Chromatin remodeling enzyme (CRE) of a Plasmodium or a related species of the Apicomplexa, Amoebozoa or Euglenozoa group, and wherein said CRE has a CRE ATPase domain. The invention also pertains to a pharmaceutical composition comprising the compound for use according to the first aspect of this invention, and a pharmaceutical acceptable carrier, stabilizer and/or excipient. The invention further relates to a pharmaceutical combination comprising: (i) the compound for use according to the first aspect of this invention, or the pharmaceutical composition according to the second aspect of this invention, and (ii) an additional anti-malarial compound. Further provided are kits comprising the compound for use according to the first aspect of this invention, the pharmaceutical composition according to the second aspect of this invention, or the pharmaceutical combination according to the third aspect of this invention, and technical instructions providing information on administration and/or dosage of the compound or the pharmaceutical composition.

### DESCRIPTION

Malaria is an acute and often chronic infectious disease caused by protozoan parasites of the genus Plasmodium. The single-celled parasites infect and destroy red blood cells, leading to fever, severe anemia, cerebral malaria and, if untreated, death. Malaria is transmitted from person to person by the bite of female mosquitos. Malaria is a disease that is prevalent in many developing countries. Approximately 40% of the world's population resides in countries where the disease is endemic, and approximately 247 million people suffer from the disease every year.

Four species of Plasmodium protozoan parasites are generally responsible for malaria, including *Plasmodium falciparum (Pf), Plasmodium vivax, Plasmodium malariae,* and *Plasmodium ovale.* Plasmodium falciparum is the dominant species in sub-Saharan Africa and is responsible for half of all clinical cases of malaria as well as 90% of deaths from the disease. The disease burden is heaviest in African children under 5 years of age and in pregnant women. Plasmodium vivax causes 25-40% of the global malaria burden, particularly in South and Southeast Asia, and Central and South America.

Malaria becomes more and more difficult to treat due to the development of resistant strains. Examples of such strains are chloroquine resistant, pyrimethamine resistant, and artemisinin resistant strains of Plasmodium falciparum.

EP3137455A1 relates to triaminopyrimidines and to pharmaceutically acceptable salts thereof, to their use in the treatment and or prevention of malaria caused by plasmodium species, and to their methods of preparation.

EP1514871A1 relates to methods for treating malaria, schistosomiasis, and cancer using dispiro 1,2,4-trioxolanes.

EP2526090A2 relates to a use of aminopyridine derivatives in the manufacture of a medicament for preventing or treating malaria.

The complex life cycle of the malaria parasite *Plasmodium falciparum (Pf)* involves the differentiation into multiple distinct forms, requiring a tight and dynamic control of gene expression at each stage. However, the full range of variation cannot solely be explained by stage-specific transcription factors, such as ApiAP2-family members, as they are strongly underrepresented in *Pf.* Huge changes in chromatin structure and epigenetic modifications during life cycle progression suggest a central role of these mechanisms in regulating the transcriptional program for parasite development. *Pf* chromatin is distinct from other eukaryotes with an extraordinarily high AT-content (>80 %) and highly divergent histones lacking typical DNA packaging properties.

The chromatin structure at promoters either restricts or enables transcription factor access, representing a crucial mechanism for gene expression regulation in eukaryotes. Histone octamers positioned on DNA are a major obstacle for sequence-specific DNA binding factors, and epigenetic mechanisms such as site-specific DNA/histone modification or RNA-mediated processes additionally regulate accessibility. Furthermore, the maintenance and re-establishment of chromatin upon transcription elongation or DNA replication requires a molecular machinery for nucleosome assembly, positioning, and spacing.

Chromatin remodeling enzymes (CREs) are ATP-dependent enzymes exerting movement, disruption or assembly of nucleosomes, the exchange of histones for variants, or generally altering chromatin structure to control access to DNA in chromatin. Histone CREs and their associated proteins are highly conserved across eukaryotes. In comparison, human cells have evolved 53 Snf2 family enzymes, potentially forming more than 1000 distinct remodeling complexes with associated proteins.

In contrast, *Plasmodium falciparum* encodes only 10 enzymes with related ATPase domains, lacking the crucial nucleosome remodeling motifs, and does not encode known complex subunits. It is unclear whether these ATPases represent functional remodeling enzymes and how they integrate into multiprotein complexes. The enzyme divergence may represent an adaption to the extraordinarily high AT-content, which averages over 90% in non-coding regions, and the highly divergent chromatin architecture, suggesting different mechanisms of *Pf* chromatin organization.

Plasmodium falciparum parasites have meanwhile become resistant almost worldwide to commonly used anti-malaria agents, such as quinine or chloroquine, and are already showing resistance to some arteminisin derivatives. In order to overcome the threat of widespread multi-drug resistant parasites, such as Plasmodium falciparum, there is an urgent need to develop new antimalarial agents particularly those with a novel mechanism of action. The present invention aims at addressing such drawbacks in the art associated with the treatment of malaria.

The inventors surprisingly identified the chromatin remodelling enzyme pfSnf2L as Imitation switch (ISWI)-related ATPase and a target of particular interest of *Plasmodium falciparum* because it has only a low homology to the closest related human chromatin remodelling enzyme and is yet essential for plasmodial survival. Therefore, targeting the chromatin remodelling enzyme pfSnf2L is promising for effectively inhibiting the formation of gametocytes of plasmodia as well as selectively killing plasmodia, such as *Plasmodium falciparum,* while not targeting a human chromatin remodelling enzyme and thus being safe for use in humans. Accordingly, targeting pfSnf2L is a promising approach for the treatment of a Malaria infection.

### BRIEF DESCRIPTION OF THE INVENTION

Generally, and by way of brief description, the main aspects of the present invention can be described as follows:

In a first aspect, the invention pertains to a compound for use in a method of diagnosis, prevention, and/or treatment of a Malaria infection in a subject, wherein the compound is an inhibitor of a Chromatin remodeling enzyme (CRE) of a Plasmodium or a related species of the Apicomplexa, Amoebozoa or Euglenozoa group, and wherein said CRE has a CRE ATPase domain.

In a second aspect, the invention pertains to a method for identifying and/or characterizing a compound suitable for the prevention and/or treatment of a disease.

In a third aspect, the invention pertains to a compound as identified by a method for identifying and/or characterizing a compound suitable for the prevention and/or treatment of a disease.

In a fourth aspect, the invention pertains to a pharmaceutical composition comprising the compound for use according to the first aspect of this invention, and a pharmaceutical acceptable carrier, stabilizer and/or excipient.

In a fifth aspect, the invention pertains to a pharmaceutical combination comprising:
(i) the compound for use according to the first aspect of this invention, or the pharmaceutical composition according to the second aspect of this invention, and
(ii) an additional anti-malarial compound.

In a sixth aspect, the invention pertains to a kit comprising the compound for use according to the first aspect of this invention, the pharmaceutical composition according to the second aspect of this invention, or the pharmaceutical combination according to the third aspect of this invention, and technical instructions providing information on administration and/or dosage of the compound or the pharmaceutical composition.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a compound for use in a method of diagnosis, prevention, and/or treatment of a Malaria infection in a subject, wherein the compound is an inhibitor of a Chromatin remodeling enzyme (CRE) of a Plasmodium, or a related species of the Apicomplexa, Amoebozoa or Euglenozoa group, and wherein said CRE has a CRE ATPase domain.

In a preferred embodiment, the compound alters the development and/or the life cycle of the Plasmodium or the related species of the Apicomplexa, Amoebozoa or Euglenozoa group, inhibits the growth of the Plasmodium or the related species of the Apicomplexa, Amoebozoa or Euglenozoa group, inhibits the survival of the Plasmodium or the related species of the Apicomplexa, Amoebozoa or Euglenozoa group, and/or kills the Plasmodium or the related species of the Apicomplexa, Amoebozoa or Euglenozoa group.

In another preferred embodiment, which can be combined with all other specifically preferred embodiments and aspects of the present invention, the compound is an inhibitor of a CRE Snf2L of the Plasmodium or a related species of the Apicomplexa, Amoebozoa or Euglenozoa group, and wherein the compound binds to the CRE Snf2L of the Plasmodium or the related species of the Apicomplexa, Amoebozoa or Euglenozoa group, optionally wherein the CRE Snf2L repositions nucleosomes in an ATP-dependent manner, and/or moves histone octamers along a nucleic acid, such as a DNA.

In one embodiment, the compound controls the gene expression in the Plasmodium, or the related species of the Apicomplexa, Amoebozoa or Euglenozoa group, via altering the positions of the nucleosomes and/or affecting the structure of chromatin.

In a specific embodiment, the compound spatiotemporally controls nucleosome positioning at promoters of stage-specific genes, and thereby controls just-in-time transcription, activation and/or repression of stage-specific genes. In a further preferred embodiment, the compound spatiotemporally controls nucleosome positioning at promoters of stage-specific genes of the Plasmodium, or the related species of the Apicomplexa, Amoebozoa or Euglenozoa group, and thereby controls just-in-time transcription, activation and/or repression of stage-specific genes of the Plasmodium, or the related species of the Apicomplexa, Amoebozoa or Euglenozoa group.

In one preferred embodiment, the compound represses gametocyte-specific gene expression, and thereby inhibits the formation of gametocytes of the Plasmodium or the related species of the Apicomplexa, Amoebozoa or Euglenozoa group, and/or prevents the initiation of gametocytogenesis in the Plasmodium or the related species of the Apicomplexa, Amoebozoa or Euglenozoa group.

In yet another embodiment, the compound according to the present invention specifically represses gametocyte-specific gene expression, and thereby specifically prevents the initiation of gametocytogenesis in the Plasmodium or the related species of the Apicomplexa, Amoebozoa or Euglenozoa group.

In one embodiment, the compound according to the present invention influences, such as specifically influences, the asexual and sexual intraerythrocytic stages of *Plasmodium falciparum* development.

In one preferred embodiment, the compound is an epigenetic modulator.

In another embodiment, the CRE ATPase domain has a sequence homology of no more than 40% to the amino acid sequence of full length human Snf2L according to SEQ ID No: 1, preferably of no more than 35%, more preferably of no more than 30%, and most preferably of around 26% sequence homology with respect to the full length human Snf2L sequence according to SEQ ID No: 1.

In a particularly preferred embodiment, which can be combined with any aspect and specific embodiment of the present invention, the compound is an inhibitor of pfSnf2L of P. falciparum.

The inventors surprisingly identified the chromatin remodelling enzyme pfSnf2L as a target of particular interest because it has only 26% homology to the closest related human chromatin remodelling enzyme and is yet essential for plasmodial survival. Therefore, targeting the chromatin remodelling enzyme pfSnf2L using a compound according to the present invention can effectively prevent the reproduction of plasmodia and kill plasmodia, while not targeting a human chromatin remodelling enzyme and thus being safe for use in humans.

Accordingly, the non-conserved (i.e. "divergent") chromatin remodelling enzyme pfSnf2L is a particularly interesting target due to the sequence divergence to human chromatin remodelling enzymes

In another preferred embodiment, which can be combined with any aspect and specific embodiment of the present invention, the compound of the present invention is directly and/or specifically targeting pfSnf2L of P. falciparum.

In one embodiment, the CRE is selected from any one of the CRE Snf2L of the Plasmodium species P. falciparum, P. vivax, P. malariae, P. ovale, P. knowlesi, P. cynomolgi, P. berghei, P. praefalciparum, P. reichenowi, P. gaboni, P. inui San Antonio 1, P. relictum SGS1-like, P. fragile, P. gallinaceum, P. vinckei vinckei, P. yoelii, and their variants, preferably having an amino acid sequence according to any one of SEQ ID Nos: 2-17.

Of note, five of the above listed species are commonly accepted as human malaria parasites: Plasmodium falciparum, Plasmodium vivax, Plasmodium malariae, Plasmodium ovale curtisi, and Plasmodium ovale wallikeri, which can be targeted using a compound according to the present invention.

Moreover, the Snf2L of the plasmodium species showing zoonotic malaria - P.knowlesi and P.cynomolgi - also show a high similarity to Snf2L of the Plasmodium species P. falciparum (pfSnf2L). Also the P. falciparum Snf2L ATPase domain exhibits a high similarity to the Snf2L ATPase domain of P. berghei, the rodent malaria Plasmodium, presumably inhibiting the enzyme of these parasites and allowing to perform subsequent toxicity and functionality studies in these rodent model systems.

The similarity of the ATPase domain of pfSnf2L of P. falciparum and the ATPase domain of Snf2L enzymes of other species is shown in Table 4. Of note, a compound targeting the ATPase domain of pfSnf2L is also highly likely to also target a similar ATPase domain of a Snf2L enzyme of other species, such as any one of the species listed in Table 4.

In yet another particularly preferred embodiment, the CRE is pfSnf2L of the Plasmodium species P. falciparum, having an amino acid sequence according to SEQ ID No: 2, optionally wherein SEQ ID NO: 2 corresponds to PF3D7_1104200.

In a further embodiment, the compound is binding to and/or is specifically inhibiting the CRE Snf2L in the Plasmodium or the related species of the Apicomplexa, Amoebozoa or Euglenozoa group.

In another preferred embodiment, the compound is 1-Benzyl-3-cetyl-2-methylimidazolium iodide (NH125) according to Formula (I)
or 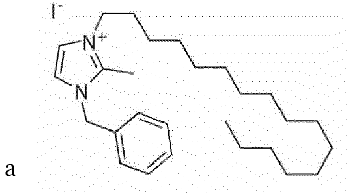 , derivative thereof.

The inventors surprisingly found that the small molecule compound 1-Benzyl-3-cetyl-2-methylimidazolium iodide (NH125) according to Formula (I) is an inhibitor of a Chromatin remodeling enzyme (CRE) of a Plasmodium, or a related species of the Apicomplexa, Amoebozoa or Euglenozoa group, such as Plasmodium falciparum. Said compound selectively binds to the remodeling enzyme Snf2L and kills Plasmodia. The use of NH125 therefore allows the selective killing of the Plasmodium, or the related species of the Apicomplexa, Amoebozoa or Euglenozoa group, such as of Plasmodium falciparum. Accordingly, the use of NH125 and/or treatment with NH125 in a patient therefore inhibits the progression of Malaria. Importantly, Snf2L drives Plasmodium just-in-time gene expression and gametocytogenesis. Accordingly, using a compound of the present invention, such as NH125 or a derivative thereof, which specifically inhibits the remodeling enzyme Snf2L, also prevents the further (sexual) development of Plasmodia.

In one embodiment, the compound is 1-Benzyl-3-cetyl-2-methylimidazolium iodide (NH125) having the CAS number 278603-08-0.

In a further preferred embodiment, the derivative of the compound of 1-Benzyl-3-cetyl-2-methylimidazolium iodide (NH125) according to Formula (I) is a molecule with similar chemical properties compared to 1-Benzyl-3-cetyl-2-methylimidazolium iodide (NH125) according to Formula (I), and wherein the derivative thereof is preferably selected from:
Benzyl(hexadecyl)dimethylazanium (Cetalkonium), Benzyldimethyltetradecylazanium (Myristalkonium), Benzyl(dodecyl)dimethylazanium (Benzododecinium), Hexadecyl(2-{[(4-methoxyphenyl)methyl](pyrimidin-2-yl)amino}ethyl)dimethylazanium (Thonzonium), 1-hexadecylpyridin-1-ium (Cetylpyridinium), {10-[dimethyl(2-{[(1R,2S,5R)-5-methyl-2-(propan-2-yl)cyclohexyl]oxy}-2-oxoethyl)azaniumyl]decyl}dimethyl(2-{[(1R,2S,5R)-5-methyl-2-(propan-2-yl)cyclohexyl]oxy}-2-oxoethyl)azanium (Decamethoxine), Hexadecyl 2-ethylhexanoate (Cetyl ethylhexanoate), Methyl 2-[1-(2-{4-[(quinolin-2-yl)methoxy]phenyl}ethyl)piperidin-4-ylidene]-4,7-diazatricyclo[8.4.0.0^{3,7}]tetradeca-1(14),3,5,10,12-pentaene-6-carboxylate (Laniquidar), 1,3-bis(heptanoyloxy)propan-2-yl heptanoate (Triheptanoin), 2-{[(2R)-1-(4-{4-[3-(azepan-1-yl)propoxy]phenyl}butyl)pyrrolidin-2-yl]methyl}-4-[(4-chlorophenyl)methyl]-1,2-dihydrophthalazin-1-one (GSK-1004723), (2S)-1-[(2S,3S)-3-hexyl-4-oxooxetan-2-yl]tridecan-2-yl (2S)-2-formamido-4-methylpentanoate (Orlistat), 2-(hexadecyloxy)-6-methyl-4H-3,1-benzoxazin-4-one (Cetilistat), (1S,3aS,3bR,9bS,11aS)-7-(heptanoyloxy)-11a-methyl-1H,2H,3H,3aH,3bH,4H,5H,9bH,10H,11H,11aH-cyclopenta[a]phenanthren-1-yl heptanoate (Estradiol dienanthate), Hexadecyltrimethylazanium (2S)-2-(6-methoxynaphthalen-2-yl)propanoate (Cetyltrimethylammonium naproxenate), Hexadecyltrimethylazanium (Cetrimonium), Diethyl(methyl)(2-{4-[2-(octyloxy)benzamido]benzoyloxy}ethyl)azanium (Otilonium), Pentadecyl octanoate (Hexadecyl Octanoate), Dodecyldimethyl(2-phenoxyethyl)azanium (Domiphen), Tert-butyl (2S)-2-[(2S)-4-(benzyloxy)-2-{[(tert-butoxy)carbonyl]amino}-4-oxobutanamido]-6-{[(benzyloxy)carbonyl]amino}hexanoate (Reversin 121), Bis(decyl)dimethylazanium (Didecyldimethylammonium), 2-{4-[3-(2-chloro-10H-phenothiazin-10-yl)propyl]piperazin-1-yl}ethyl heptanoate (Perphenazine enanthate), N,N,N-trimethyl-3-{[methyl({10-[methyl({[3-(trimethylazaniumyl)phenoxy]carbonyl})amino]decyl})carbamoyl]oxy}anilinium (Demecarium), N-[2-(4-{3-[(propan-2-yl)amino]pyridin-2-yl}piperazine-1-carbonyl)-1H-indol-5-yl]methanesulfonamide (Delavirdine), S-{2-[(2-chloro-4-sulfamoylphenyl)amino]-2-oxoethyl} 6-methyl-3,4-dihydroquinoline-1(2H)-carbothioate, 2,6,10,15,19,23-hexamethyltetracosane (Squalane), and N-{3-[(2-{[4-(4-methylpiperazin-1-yl)phenyl]amino}thieno[3,2-d]pyrimidin-4-yl)oxy]phenyl}prop-2-enamide (Olmutinib)

In a preferred embodiment, the compound for use in a method of diagnosis, prevention, and/or treatment of a Malaria infection in a subject according to the first aspect of this invention, which can be combined with any other aspect and specific embodiment of this invention, is a compound selected from any one of the following compounds as depicted in Table 1:

| **IUPAC Name** | **Chemical Formula** | **SMILES** | **DrugBank Accession Number; Score in swissimilarity** |
|---|---|---|---|
| (Generic Name) | | | |
| **1-Benzyl-3-cetyl-2-methylimidazo lium iodide** | 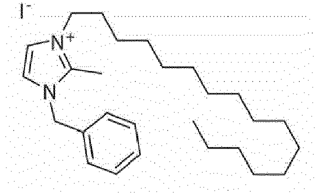 | **CCCCCCCCCCCCCCCCN₁ C=C[N+](=C₁C)CC₂=CC= CC=C2.[I-]** | |
| (NH125) | | | |
| **Benzyl(hexade cyl)dimethylaz anium** | 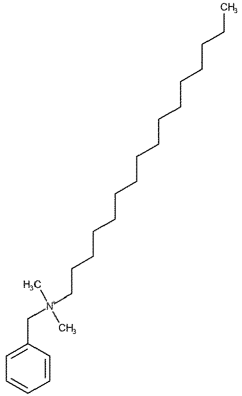 | CCCCCCCCCCCCCCCC[N+]( C)(C)CC₁=CC=CC=C₁ | DB11583; 0.387 |
| (Cetalkonium) | | | |
| **Benzyldimethy ltetradecylazan ium** | 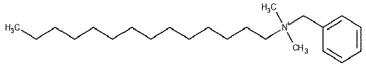 | CCCCCCCCCCCCCC[N+](C)( C)CC₁=CC=CC=C₁ | DB13774; 0.357 |
| (Myristalkonium) | | | |
| **Benzyl(dodecyl )dimethylazani um** | 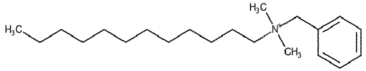 | CCCCCCCCCCCC[N+](C)(C) CC₁=CC=CC=C₁ | DB13282; 0.319 |
| (Benzododeciniu m) | | | |
| **Hexadecyl(2-{[(4-methoxyphenyl )methyl](pyri midin-2-yl)amino}ethyl )dimethylazani um** | 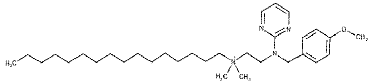 | CCCCCCCCCCCCCCCC[N+]( C)(C)CCN(CC₁=CC=C(OC)C= C₁)C₁=NC=CC=N₁ | DB09552; 0.192 |
| (Thonzonium) | | | |
| **1-hexadecylpyrid in-1-ium** | 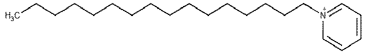 | CCCCCCCCCCCCCCCC[N+]₁ =CC=CC=C₁ | DB11073; 0.112 |
| (Cetylpyridinium) | | | |
| **{10-[dimethyl(2-{[(1R,2S,5R)-S-methyl-2-(propan-2-yl)cyclohexyl]o xy}-2-oxoethyl)azani umyl]decyl}di methyl(2-{[(1R,2S,5R)-S-methyl-2-(propan-2-yl)cyclohexyl]o xy}-2-oxoethyl)azani** | 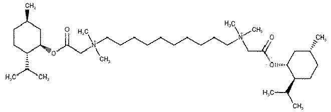 | CC(C)[C@@H]₁CC[C@@H]( C)C[C@H]1OC(=O)C[N+](C) (C)CCCCCCCCCC[N+](C)(C) CC(=O)O[C@@H]1C[C@H]( C)CC[C@H]1C(C)C | DB13705; 0.079 |
| **um** | | | |
| (Decamethoxine) | | | |
| **Hexadecyl 2-ethylhexanoate** | 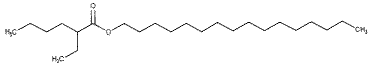 | CCCCCCCCCCCCCCCCOC(= O)C(CC)CCCC | DB11349; 0.062 |
| (Cetyl ethylhexanoate) | | | |
| **Methyl 2-[1-(2-{4-[(quinolin-2-yl)methoxy]ph enyl}ethyl)pipe ridin-4-ylidene]-4,7-diazatricyclo[8 .4.0.0^{3,7}]te tradeca-1(14),3,5,10,12-pentaene-6-carboxylate** | 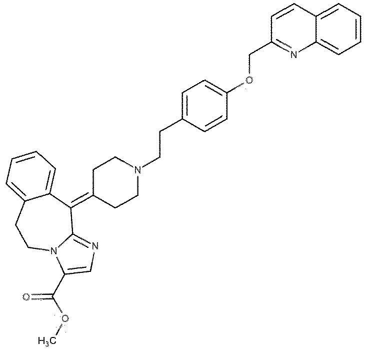 | COC(=O)C1=CN=C2N1CCC1 =CC=CC=C1C2=C1CCN(CCC 2=CC=C(OCC3=NC4=CC=C C=C4C=C3)C=C2)CC1 | DB12799; 0.060 |
| (Laniquidar) | | | |
| **1,3-bis (heptanoylo xy)propan-2-yl heptanoate** | 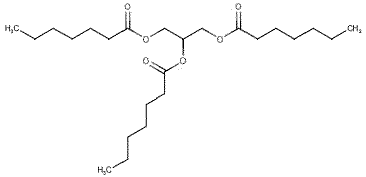 | CCCCCCC(=O)OCC(COC(=O )CCCCCC)OC(=O)CCCCCC | DB11677; 0.053 |
| (Triheptanoin) | | | |
| **2-{[(2R)-1-(4-{4-[3-(azepan-1-yl)propoxy]phe nyl}butyl)pyrr olidin-2-yl]methyl}-4-[(4-chlorophenyl) methyl]-1,2-dihydrophthal azin-1-one** | 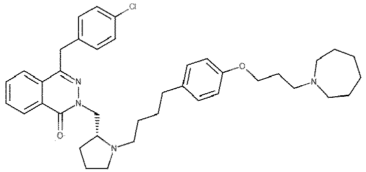 | ClC1=CC=C(CC2=NN(C[C@ H]3CCCN3CCCCC3=CC=C(O CCCN4CCCCCC4)C=C3)C(= O)C3=CC=CC=C23)C=C1 | DB12806; 0.048 |
| (GSK-1004723) | | | |
| **(2S)-1-[(2S,3S)-3-hexyl-4-oxooxetan-2-yl]tridecan-2-yl (2S)-2-formamido-4-methylpentano ate** | 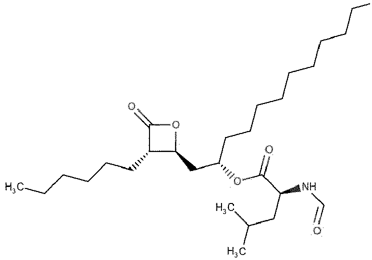 | CCCCCCCCCCC[C@@H](C[C @@H]1OC(=O)[C@H]1CCCC CC)OC(=O)[C@H](CC(C)C)N C=O | DB01083; 0.048 |
| (Orlistat) | | | |
| **2-**(hexadecyloxy) **-6-methyl**-**4H-3,1-benzoxazin-4-**one | 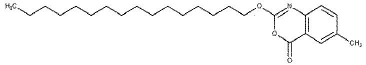 | CCCCCCCCCCCCCCCCOC1= NC2=CC=C(C)C=C2C(=O)O1 | DB06586; 0.047 |
| (Cetilistat) | | | |
| **(1S,3aS,3bR,9b S,11aS)-7-(heptanoyloxy) -11a-methyl-1H,2H,3H,3aH, 3bH,4H,5H,9b H,10H,11H,11a H-cyclopenta[a]p henanthren-1-yl heptanoate** | 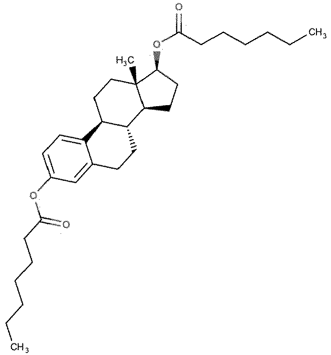 | CCCCCCC(=O)O[C@H]1CC[ C@H]2[C@@H]3CCC4=CC( OC(=O)CCCCCC)=CC=C4[C @H]3CC[C@]12C | DB13955; 0.046 |
| (Estradiol dienanthate) | | | |
| **Hexadecyltrim ethylazanium (2S)-2-(6-methoxynapht halen-2-yl)propanoate** | 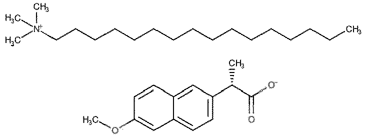 | CCCCCCCCCCCCCCCC[N+]C | DB15877; 0.044 |
| (Cetyltrimethyla mmonium naproxenate) | | | |
| **Hexadecyltrim ethylazanium** | 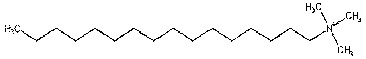 | COC1=CC2=CC=C(C=C₂C=C 1)[C@H](C)C([O-])=O.CCCCCCCCCCCCCCCC[ N+](C)(C)C | DB01718; 0.044 |
| (Cetrimonium) | | | |
| **Diethyl(methyl )(2-{4-[2-(octyloxy)benz amido]benzoyl oxy}ethyl)azan ium** | 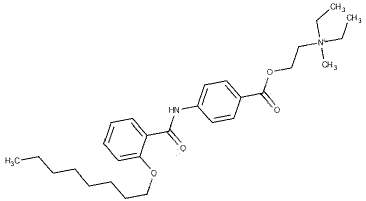 | CCCCCCCCOC1=CC=CC=C1C (=O)NC1=CC=C(C=C1)C(=O) OCC[N+](C)(CC)CC | DB13500; 0.043 |
| (Otilonium) | | | |
| Pentadecyl octanoate | 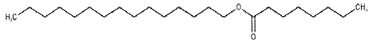 | CCCCCCCCCCCCCCCOC(=O) CCCCCCC | DB02425; 0.041 |
| (Hexadecyl Octanoate) | | | |
| **Dodecyldimeth yl(2-phenoxyethyl)a zanium** | 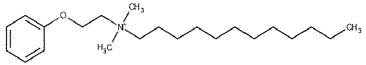 | CCCCCCCCCCCC[N+](C)(C) CCOC1=CC=CC=C1 | DB11594; 0.038 |
| (Domiphen) | | | |
| **Tert-butyl (2S)-2-[(2S)-4-(benzyloxy)-2-{[(tert-butoxy) carbon yl]amino}-4-oxobutanamid o]-6-{[(benzyloxy)c arbonyl] amino }hexanoate** | 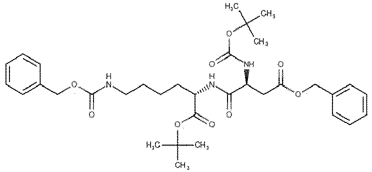 | CC(C)(C)OC(=O)N[C@@H]( CC(=O)OCC1=CC=CC=C1)C( =O)N[C@@H](CCCCNC(=O) OCC1=CC=CC=C1)C(=O)OC( C)(C)C | DB14072; 0.036 |
| (Reversin 121) | | | |
| **Bis(decyl)dime thylazanium** | 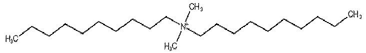 | CCCCCCCCCC[N+](C)(C)CC CCCCCCCC | DB04221; 0.036 |
| (Didecyldimethyl ammonium) | | | |
| **2-{4-[3-(2-chloro-10H-phenothiazin-10-yl)propyl]piper azin-1-yl}ethyl heptanoate** | 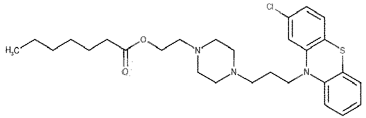 | CCCCCCC(=O)OCCN1CCN(C CCN2C3=CC=CC=C3SC3=C2 C=C(Cl)C=C3)CC1 | DB14651; 0.029 |
| (Perphenazine enanthate) | | | |
| **N,N,N-trimethyl-3-{[methyl({10-[methyl({[3-(trimethylazan iumyl)phenoxy ]carbonyl})ami no]decyl})carb amoyl] oxy}anil inium** | 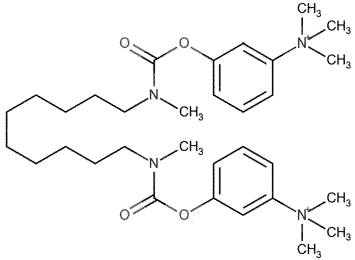 | CN(CCCCCCCCCCN(C)C(=O) OC1=CC=CC(=C1)[N+](C)(C) C)C(=O)OC1=CC=CC(=C1)[N +](C)(C)C | DB00944; 0.028 |
| (Demecarium) | | | |
| **N-[2-(4-{3-[(propan-2-yl)amino]pyrid in-2-yl}piperazine-1-carbonyl)-1H-indol-5-yl]methanesulf onamide** | 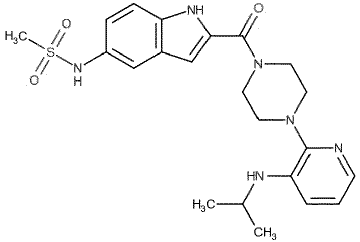 | CC(C)NC1=CC=CN=C1N1CC N(CC1)C(=O)C1=CC2=CC(N S(C)(=O)=O)=CC=C2N1 | DB00705; 0.026 |
| (Delavirdine) | | | |
| **S-{2-[(2-chloro-4-sulfamoylphen yl)amino]-2-oxoethyl} 6-methyl-3,4-dihydroquinoli ne-1(2H)-carbothioate** | 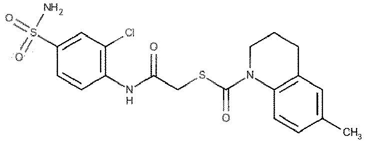 | CC1=CC2=C(C=C1)N(CCC2) C(=O)SCC(=O)NC1=C(Cl)C= C(C=C1)S(N)(=O)=O | DB08494; 0.021 |
| **2,6,10,15,19,23-hexamethyltetr acosane** | 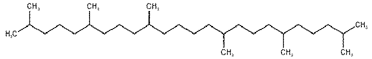 | CC(C)CCCC(C)CCCC(C)CCCC C(C)CCCC(C)CCCC(C)C | DB11420; 0.014 |
| (Squalane) | | | |
| **N-{3-[(2-{[4-(4-methylpiperazi n-1-yl)phenyl] amin o}thieno [3,2-d]pyrimidin-4-yl)oxy]phenyl} prop-2-enamide** | 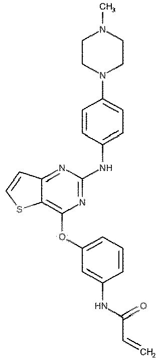 | CN1CCN(CC1)C1=CC=C(NC2 =NC(OC3=CC=CC(NC(=O)C =C)=C3)=C3SC=CC3=N2)C= C1 | DB13164; 0.011 |
| (Olmutinib) | | | |

According to the present invention, the score in swissimilarity (as depicted in the last column of Table 1) is a combined score that is calculated from similarity values obtained using two different molecular representations: FP2 fingerprints (2D method) and ES5D vector (3D method). Mathematically, the combined score is a probability that a given couple of molecules share a common protein target. It was obtained using logistic regression with two features:
f(FP2,ES5D)=(1+exp(-a0-a1FP₂-a2ES5D))-1, where:
- FP₂ and ES5D are similarities between FP₂ fingerprints and ES5D vectors, respectively;
- a0, a1 and a2 are parameters learned by the model to predict possible protein targets for a small molecule based on molecular similarity to known bioactive compounds.

In one embodiment, the compound for use in a method of diagnosis, prevention, and/or treatment of a Malaria infection in a subject according to the first aspect of this invention, which can be combined with any other aspect and specific embodiment of this invention, is a compound having a swissimilarity as depicted in the last column of Table 1.

According to the present invention, any type of Malaria infection can be treated with a compound for use according to the first aspect of the present invention. In a preferred embodiment, the Malaria infection is an uncomplicated, a severe complicated, a cerebral, and/or a multi-drug resistant malaria infection, such as a chloroquine resistant malaria infection, a mefloquine resistant malaria infection, a mapaerine resistant malaria infection, a quinine resistant malaria infection, an amidoquine resistant malaria infection, and/or a halofantrine resistant malaria infection.

Importantly, Plasmodium falciparum has meanwhile become resistant almost worldwide to commonly used anti-malaria agents, such as quinine or chloroquine. Plasmodium falciparum parasites are further already showing resistance to some arteminisin derivatives. Accordingly, new effective drugs against malaria, such as Malaria caused by a protozoan parasite, such as Plasmodium falciparum, are desperately needed.

In a further preferred embodiment, the Malaria infection is Malaria tropica, Malaria tertiana, and/or Malaria quartana.

In one embodiment, the method comprises the administration of the compound to a subject, optionally wherein the administration is by oral, interactal, intramuscular, subcutaneous, intradermal, intra-peritoneal, intravenous, and/or intra-pleural route, further optionally wherein the subject is a human, a monkey, a mouse, a rat, a rabbit, a reptile, a bird, a bat, a lizard, or an antelope, preferably wherein the subject is a patient, such as a comatose patient.

In a second aspect, the invention pertains to a method for identifying and/or characterizing a compound suitable for the prevention and/or treatment of a disease, the method comprising the steps of:
a) Providing at least one parasite, such as a Plasmodium or a related species of the Apicomplexa, Amoebozoa or Euglenozoa group, and a candidate compound, wherein the parasite comprises at least one CRE Snf2L;
b) Bringing into contact the at least one parasite and the candidate compound, and
c) Detecting and/or quantifying the level of activity of the at least one CRE Snf2L, optionally wherein the detecting and/or quantifying the level of activity of the at least one CRE Snf2L comprises measuring the reduction of an ATPase activity of the at least one CRE Snf2L, further optionally wherein the detecting and/or quantifying the level of activity of the at least one CRE Snf2L does not comprise monitoring the basal ATPase activity of the at least one CRE Snf2L;
wherein a reduction and/or inhibition of the level of activity of the at least one CRE Snf2L of the at least one parasite contacted with the candidate compound compared to the activity of the at least one CRE Snf2L of the at least one parasite not contacted with the candidate compound indicates the candidate compound as suitable for the prevention and/or treatment of the disease.

Optionally, the method according to the second aspect of the present invention further comprises the step of providing nucleoprotein complexes (i.e. a combination of DNA, nucleosomes, histones, histone peptides, and peptides encompassing the histone H4 N-terminal domain).

The method according to the second aspect of the present invention involves a screening that measures the loss of ATPase activity of the substrate stimulated CRE. Of note, CREs require specific nucleoprotein complexes (i.e. a combination of DNA, nucleosomes, histones, histone peptides, and peptides encompassing the histone H4 N-terminal domain) as substrates for their functional activity and active ATPase cycles. The screening system invokes the functional active substrate cycle for testing inhibitors and does not monitor the basal ATPase activity of the CRE.

In a preferred embodiment relating to the second aspect of the present invention, the disease is a Malaria. In a further preferred embodiment, the Malaria infection is an uncomplicated, a severe complicated, a cerebral, and/or a multi-drug resistant malaria infection, such as a chloroquine resistant malaria infection, a mefloquine resistant malaria infection, a mapaerine resistant malaria infection, a quinine resistant malaria infection, an amidoquine resistant malaria infection, and/or a halofantrine resistant malaria infection, optionally wherein the Malaria infection is Malaria tropica, Malaria tertiana, and/or Malaria quartana.

In a third aspect, the invention pertains to a compound as identified by a method for identifying and/or characterizing a compound suitable for the prevention and/or treatment of a disease according to the second aspect of this invention.

According to the present invention, any embodiment of the first aspect of this invention can be combined with the second and third aspect of this invention.

In one embodiment, a compound as identified by a method for identifying and/or characterizing a compound suitable for the prevention and/or treatment of a disease according to the second aspect of this invention (i.e. a compound according to the third aspect of this invention) is a compound for use according to the first aspect of this invention.

In a fourth aspect, the invention pertains to a pharmaceutical composition comprising the compound for use according to the first aspect of this invention, or a compound according to the third aspect of this invention, and a pharmaceutical acceptable carrier, stabilizer and/or excipient.

In a fifth aspect, the invention pertains to a pharmaceutical combination comprising:
(i) the compound for use according to the first aspect of this invention, a compound according to the third aspect of this invention, or the pharmaceutical composition according to the fourth aspect of this invention, and
(ii) an additional anti-malarial compound.

The pharmaceutical combination according to the present invention can comprise any kind of anti-malarial compound as additional anti-malarial compound under (ii). In one embodiment, the additional anti-malarial compound is selected from Chloroquine, Mefloquine, Mapaerine, Quinine, Amidoquine, Tafenoquine, Pyrimethamine, Lumefantrine, Hydroxychloroquine, Sulfisoxazole, Artenimol, Clotrimazole, Diethyltoluamide, Clindamycin, Lumefantrine co-artemether, Artesunate, Sulfametopyrazine, Dapsone, Quinidine, Demeclocycline, Hydralazine, Azithromycin, Sulfadoxine, Artemotil, Primaquine, Artemether, Proguanil, Doxycycline, Ciprofloxacin, Methylene blue, Acetarsol, Acetyl sulfisoxazole, Midazolam, UCB7362, PEV3A, Cipargamin, Quercetin, Artefenomel, SJ-733, Pyronaridine, ACT-451840, Artemisinin, p-Quaterphenyl, Temefos, DSM-265, Chlorproguanil, AQ-13, Transfluthrin, Artemisone, 16-Bromoepiandrosterone, Fenpyroximate, Pafuramidine, Ganaplacide, Voacamine, QS-21, JPC-3210, and Halofantrine.

In one embodiment, the pharmaceutical combination comprises one of the following additional anti-malarial compounds under (II) of the pharmaceutical combination, as listed in Table 2:

| **Generic Name of the compound** | **DrugBank Accession Number** |
|---|---|
| Tafenoquine | DB06608 |
| Pyrimethamine | DB00205 |
| Lumefantrine | DB06708 |
| Hydroxychloroquine | DB01611 |
| Sulfisoxazole | DB00263 |
| Artenimol | DB11638 |
| Clotrimazole | DB00257 |
| Diethyltoluamide | DB11282 |
| Clindamycin | DB01190 |
| Quinine | DB00468 |
| Lumefantrine co-artemether | DB13085 |
| Artesunate | DB09274 |
| Sulfametopyrazine | DB00664 |
| Chloroquine | DB00608 |
| Dapsone | DB00250 |
| Quinidine | DB00908 |
| Demeclocycline | DB00618 |
| Hydralazine | DB01275 |
| Azithromycin | DB00207 |
| Sulfadoxine | DB01299 |
| Mefloquine | DB00358 |
| Artemotil | DB13851 |
| Primaquine | DB01087 |
| Halofantrine | DB01218 |
| Artemether | DB06697 |
| Proguanil | DB01131 |
| Doxycycline | DB00254 |
| Ciprofloxacin | DB00537 |
| Methylene blue | DB09241 |
| Acetarsol | DB13268 |
| Acetyl sulfisoxazole | DB14033 |
| Midazolam | DB00683 |
| UCB7362 | DB17096 |
| PEV3A | DB05144 |
| Cipargamin | DB12306 |
| Quercetin | DB04216 |
| Artefenomel | DB11809 |
| SJ-733 | DB12659 |
| Pyronaridine | DB12975 |
| ACT-451840 | DB13054 |
| Artemisinin | DB13132 |
| p-Quaterphenyl | DB12794 |
| Temefos | DB12231 |
| DSM-265 | DB12397 |
| Chlorproguanil | DB12314 |
| AQ-13 | DB17749 |
| Transfluthrin | DB15117 |
| Artemisone | DB16097 |
| 16-Bromoepiandrosterone | DB05107 |
| Fenpyroximate | DB16164 |
| Pafuramidine | DB06532 |
| Ganaplacide | DB16173 |
| Voacamine | DB04877 |
| QS-21 | DB05400 |
| JPC-3210 | DB15609 |

In a sixth aspect, the invention pertains to a kit comprising the compound for use according to the first aspect of this invention, the compound according to the third aspect of this invention, the pharmaceutical composition according to the fourth aspect of this invention, or the pharmaceutical combination according to the fifth aspect of this invention, and technical instructions providing information on administration and/or dosage of the compound or the pharmaceutical composition.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

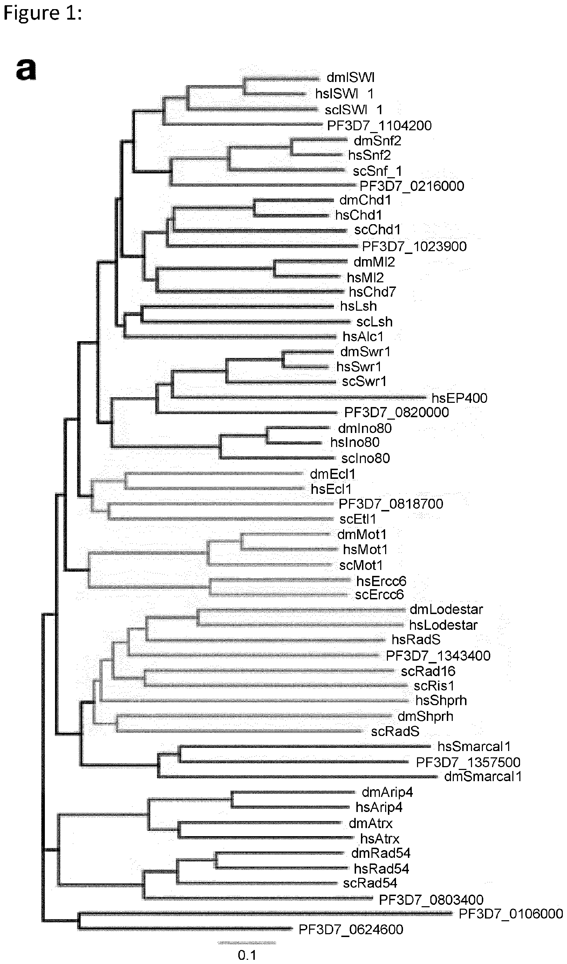

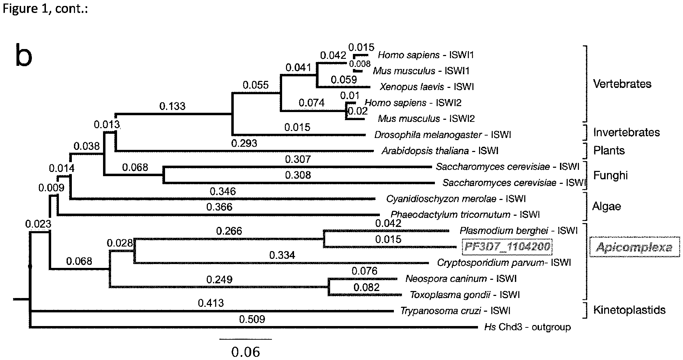

**Figure 1****: shows phylogenetic analysis of the helicase region of SWI/SNF.** Protein sequences used for in silico analysis are listed in Table 3. **a,** Phylogenetic analysis of the helicase region with subfamily representatives of Hs, Dm and Sc and predicted Pf SWI2/SNF2-ATPases, coloring represents SWI/SNF subgroups according to 1. **b,** Rooted phylogenetic tree of the full length Imitation switch (ISWI) homologues from different organisms representing eukaryotic (sub) kingdoms with Apicomplexa highlighted (blue) and HsChd3 as outgroup. Numbers indicating substitutions per site.

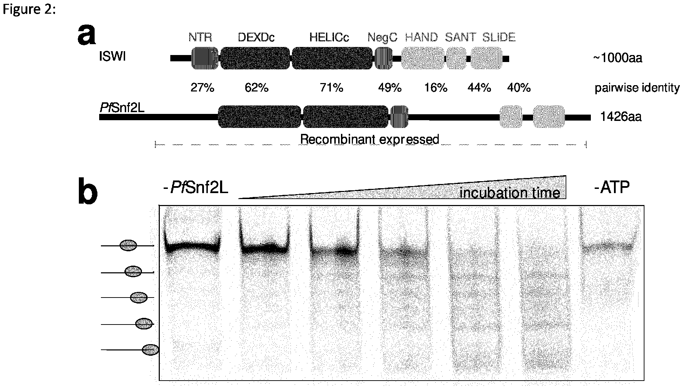

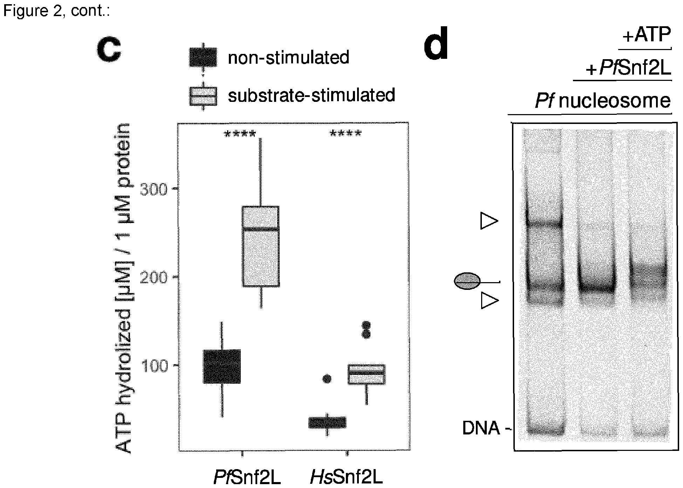

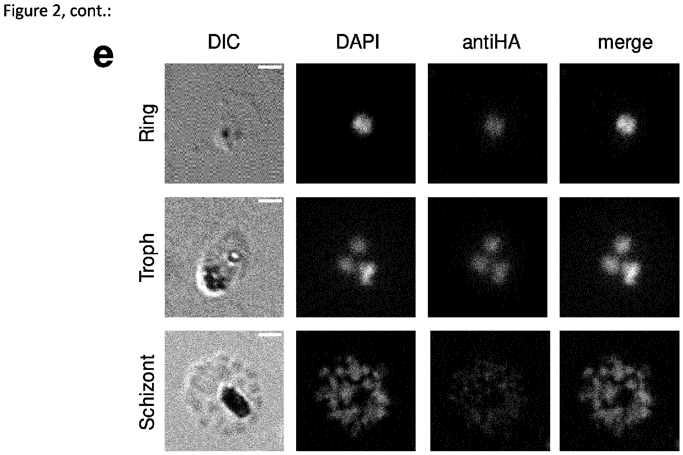

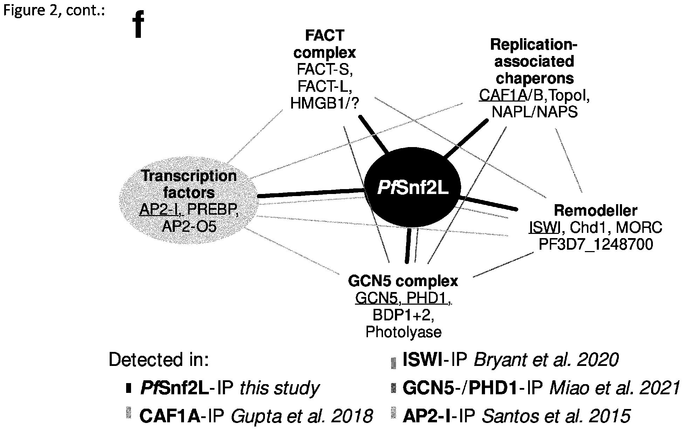

**Figure 2****: shows Enzymatic activity and interaction partners of the divergent chromatin remodeler PfSnf2L. a,** Comparison of ISWI domain architecture with PfSnf2L. The location of ATPase region (dark red), autoregulatory domains (light red) and HAND-SANT-SLIDE (grey) and their conservation are indicated. **b,** Nucleosome remodeling assay using recombinant PfSnf2L and centered-positioned nucleosomes. Relative positions of nucleosomes are indicated. **c,** Boxplot showing the non-stimulated or nucleosome-stimulated ATP hydrolysis rate of PfSnf2L/HsSnf2L (n=18/n=10 independent experiments, *two-tailed Student's t-test, unpaired,* *****p<0.001).* **d,** Nucleosome assembly assay using PfSnf2L and canonical histones. Nucleosomes and uncharacterized histone-DNA-complexes (triangle) are indicated. **e,** PfSnf2L-HA colocalization IFAs in asexual blood stages (DIC: differential interference contrast, DAPI: 4',6-diamidino-2-phenylindole, scale bar: 2 µm). **f,** PfSnf2L interaction network with proteins identified by antiHA-IP LC-MS/MS and additional interactions detected in complementary IP experiments (underlined) with corresponding colors.

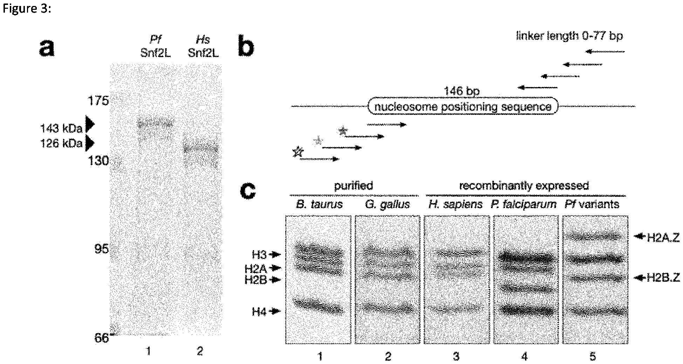

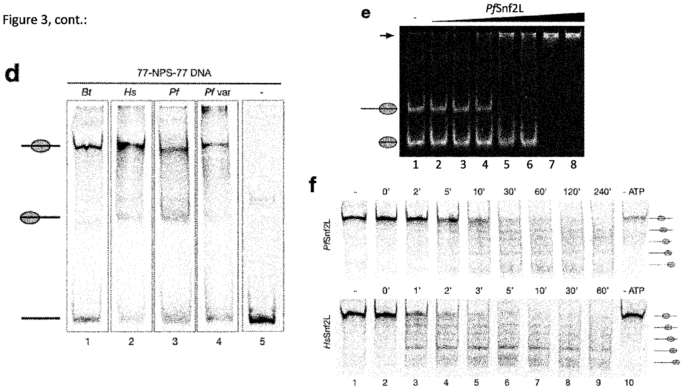

**Figure 3****: shows supplements to in vitro tools and assays, a,** SDS-PAGE (6 %, Coomassie-stained) of recombinantly expressed and purified remodeling enzymes. Expected protein band and its size is indicated. **b,** Schematic representation of DNA template generation for nucleosome assembly containing various linker lengths and fluorescence labelling (stars) implemented via PCR. **c,** SDS-PAGE (15%, Coomassie-stained) of histone octamer preparation of purified or recombinantly expressed origin. **d,** Native PAGE with reconstituted nucleosomes on Cy5-labelled 77-NPS-77 DNA (lane 5) using different octamers an in c. Relative position of nucleosome and DNA are indicated. **e,** Competitive nucleosome binding assay using *Pf*Snf2L (0.02-0.6 µM) and an equimolar mixture of *Gg*/6-NPS-47-Cy5 (red) and *Gg*/o-NPS-o-Cy3 (green) nucleosomes. Merged channels are shown with nucleosome positions and shift products (arrow) are indicated. **f,** Nucleosome remodeling assay using *Pf*Snf2L or *HsS*nf2L and centered-positioned nucleosomes (*Bt*/77-NPS-77-Cy5) as in Fig. 1b. Relative positions of nucleosomes are indicated.

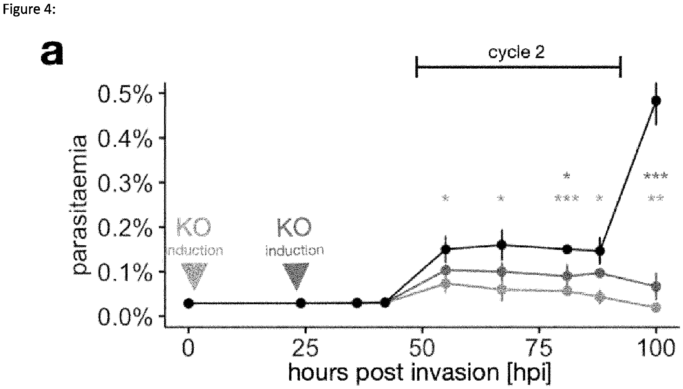

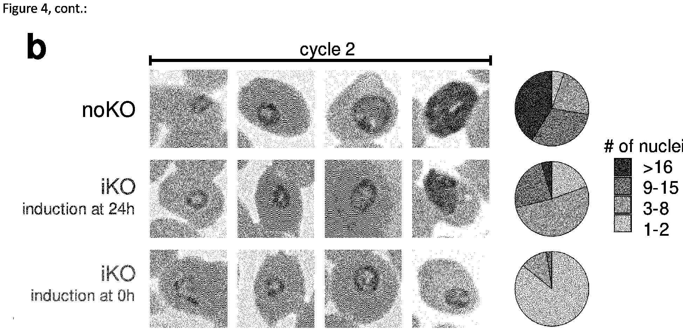

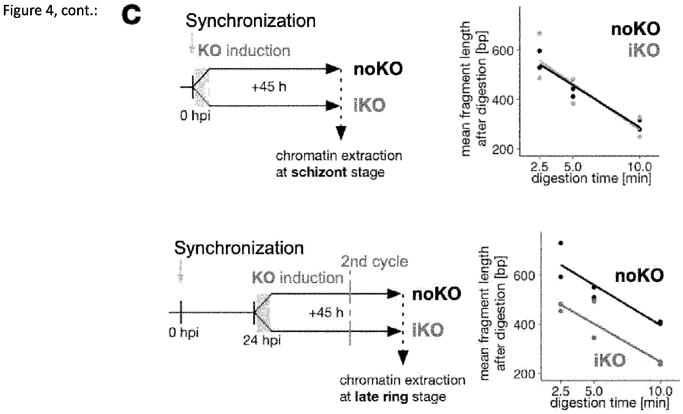

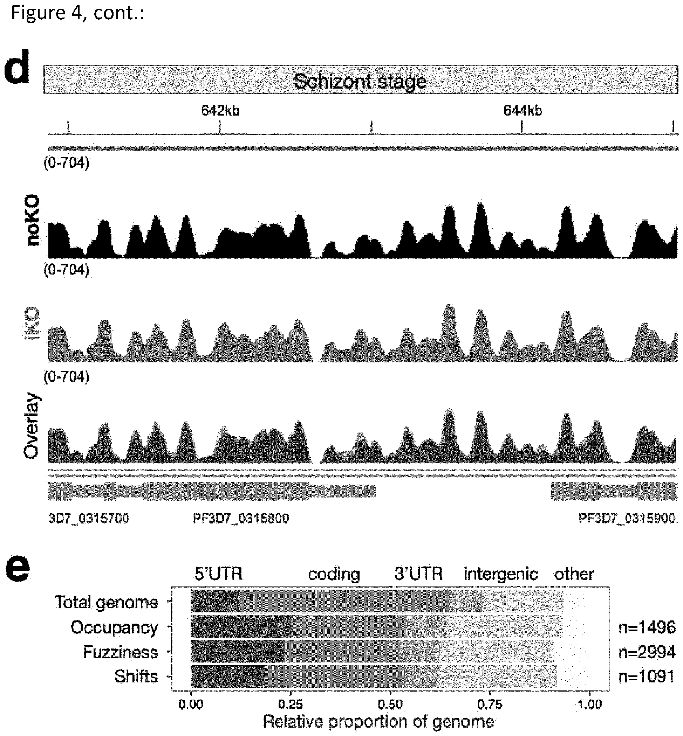

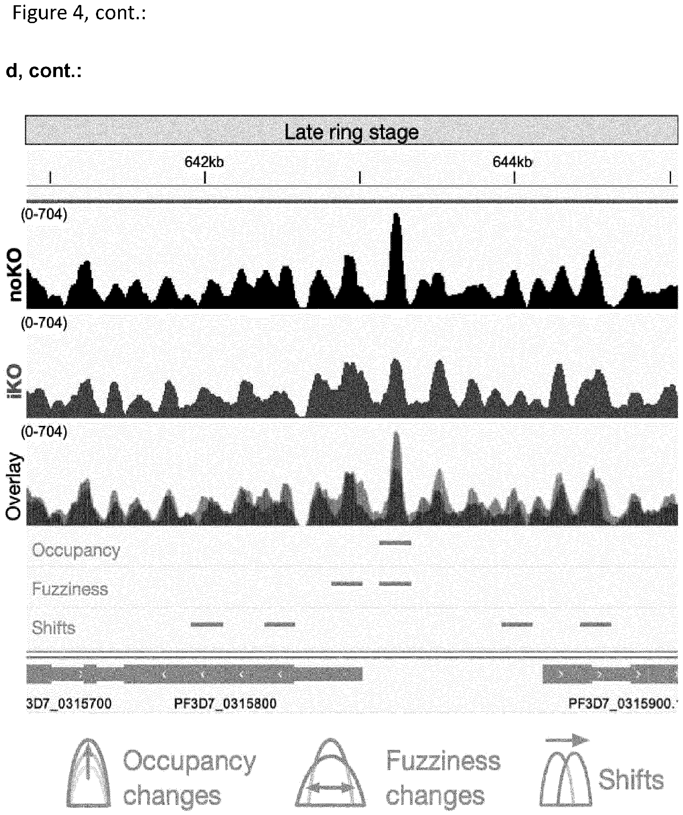

**Figure 4****: shows *Pf*Snf2L -KO results in chromatin structure changes in ring stage, preventing parasite development and viability, a,** Growth curve analysis of synchronized *PfSnf₂L-*HA-KO parasites not-induced (black), induced at 24 hpi (dark red) and induced at o hpi (light red). Mean ± SD and significance are shown (two-tailed Student's t-test, unpaired, ns p>0.05, * p<0.05, ** p<0.01, ***p<0.005). **b,** Representative Giemsa-stained blood smears of parasites treated as in a. Pie charts show number of nuclei at the end of cycle 2 (n=100 each). **c,** Scheme of KO induction and chromatin extraction at schizont (left) and late ring stage (right). The MNase digestion kinetics is presented accordingly. **d,** Exemplary coverage plots of MNase-Seq of schizont/late ring parasites upon *PfSnf₂L-*KO*.* Identified changes in occupancy, fuzziness and shifts are indicated. Regions shown is chr3:641000-645000 with annotated genes marked in grey. **e,** Assignment of *PfSnf2L-*KO dependent nucleosome positioning changes relative to functional elements of the genome.

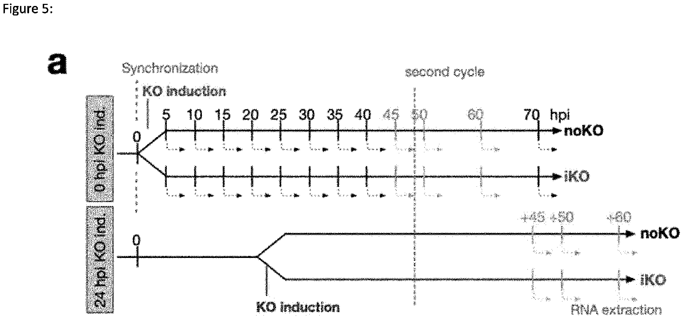

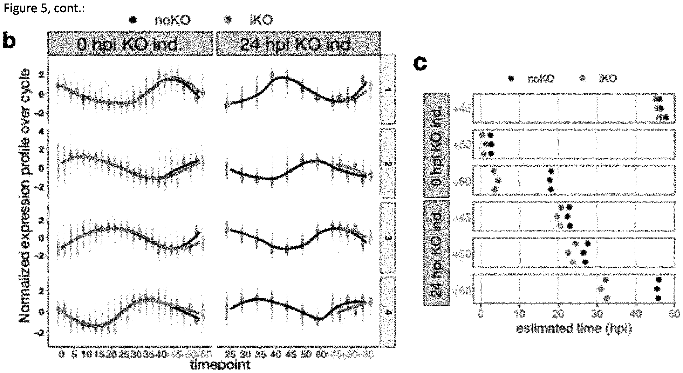

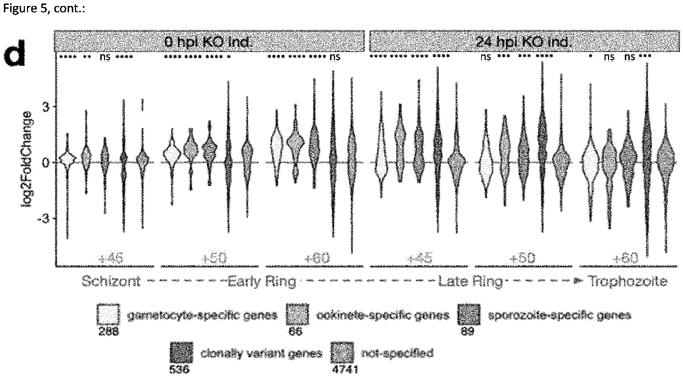

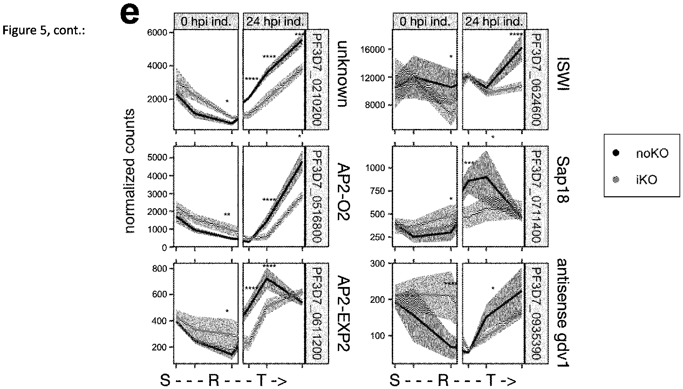

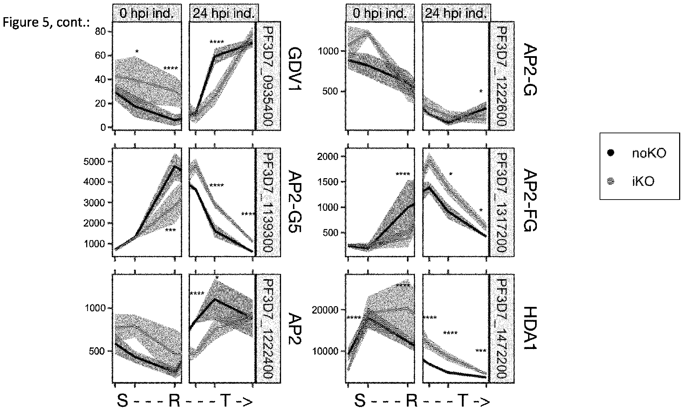

**Figure 5****: shows supplements to RNA-Seq analysis:** *Pf*Snf2L-HA-KO parasites non-induced (noKO), induced (iKO) at 0/24 hpi +45/+50/+60 hours, respectively, **a,** Experimental setup of *Pf*Snf2L-KO timing and sampling for transcriptome analysis **b,** Normalized relative expression profile of genes differentially expressed over all timepoints in noKO/iKO, clustered according to expression profile over cycle. **c,** Cycle progression estimation of noKO/iKO RNA-seq samples as in a. **d,** Expression change - log2(iKO/noKO) - at individual timepoints for stage-specific gene groups. **e,** Gene expression profiles (DESeq2 normalized) over individual timepoints of exemplary epigenetic regulators involved in gametocyte commitment.

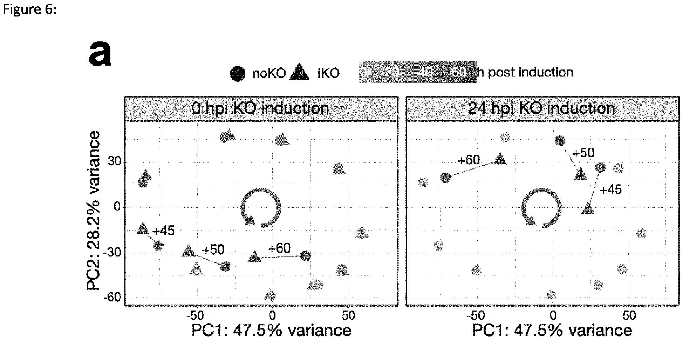

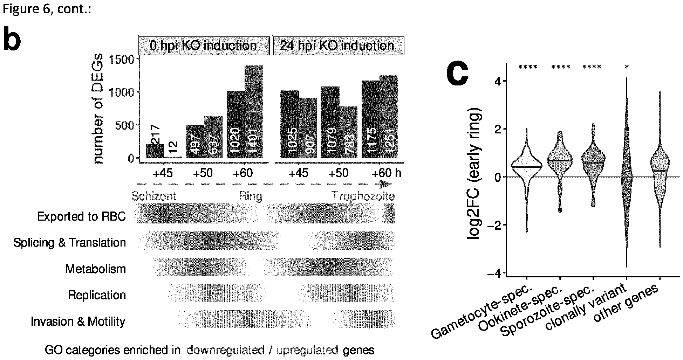

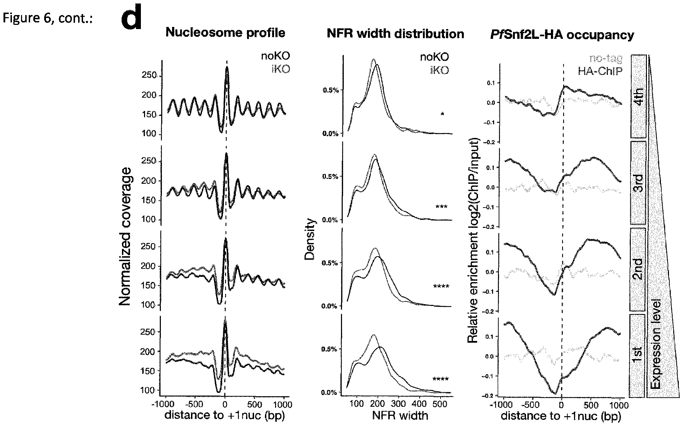

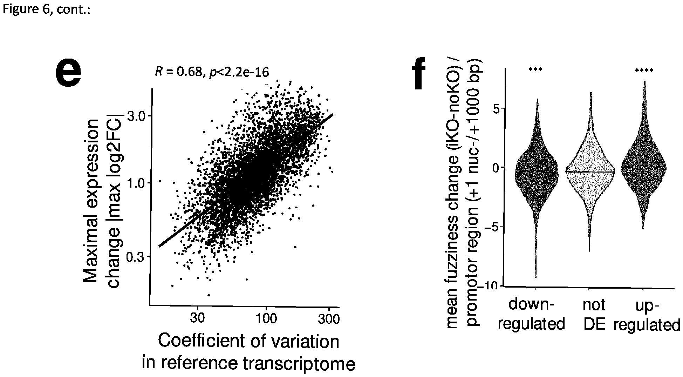

**Figure 6****: shows *Pf*Snf2L regulates just-in-time transcription by shaping promoter architecture of stage-specific genes, a,** Principal component analysis of whole RNA-seq data of highly synchronous noKO (circle) / iKO (triangle) parasites at different time points (45 h / 50 h / 60 h) post KO induction at h (left) or 24 h (right). **b,** Barplot showing numbers of differentially expressed genes (DEGs) (top) and schematic representation of superordinate GO categories enriched in DEGs (bottom) over time in iKO/noKO parasites as in a. **c,** Violin plot showing expression changes (log₂(iKO/noKO)) of stage-specific gene groups **d,** MNase-seq occupancy profiles of late ring stage iKO/noKO around the +1 nucleosome (left panel), NFR width distribution (mid panel) and *PfSnf2L* enrichment (ChIP-Seq at 34 hpi, right panel), subdivided by gene expression quantiles **e,** Pearson correlation between maximal expression change of each gene and its expression variation in the reference transcriptome. **f,** Violin plot depicting the mean nucleosome fuzziness difference (iKO-noKO) of all promoter regions (+/-1000 bp around the +1 nucleosome) with respect to differential expression, c-f, (two-tailed Student's t-test, unpaired, ns p>0.05, * p<0.05, ** p<0.01, ***p<0.005, ****p>0.001)

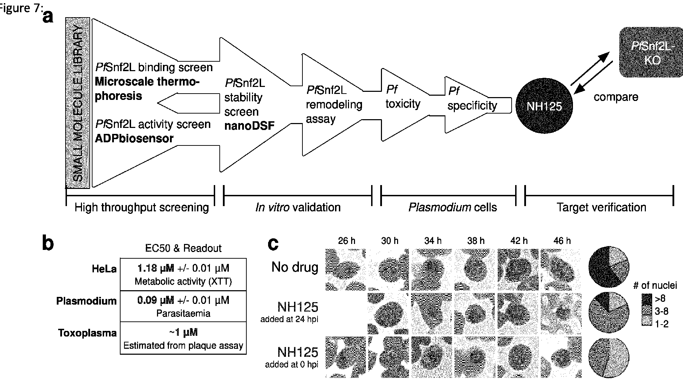

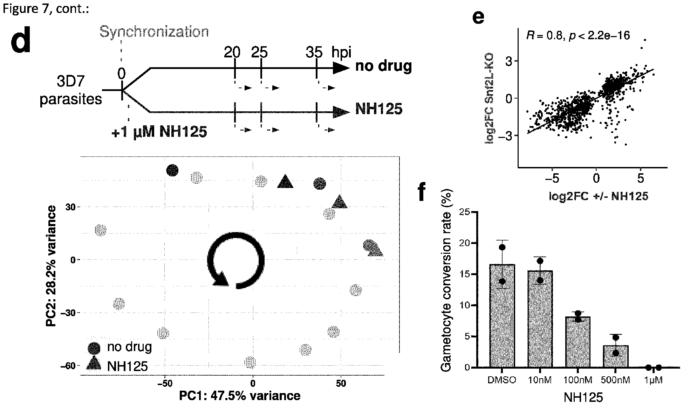

**Figure 7****: shows the potent drug NH125 specifically inhibits *Pf*Snf2L and phenocopies KO effects. a,** Scheme of the inhibitor screening pipeline **b,** NH125 toxicity assay with HeLa/Plasmodium/Toxoplasma cells. EC50 values and the corresponding readout methods are given. **c,** Representative Giemsa-stained blood smears of parasites cultured in the presence of 1 µM NH125 added at 0/24 hpi. Pie charts show number of nuclei at the end of the cycle (n>80 each). **d,** Scheme of drug treatment and RNA extraction timing (top). Principal component analysis (bottom) of whole transcriptome data (blue) and reference transcriptome (grey, as in Fig. 3a). **e,** Pearson correlation between expression change in trophozoites upon *PfSnf2L-*KO and NH125 treatment for DEGs (p<0.01). **f,** Gametocyte conversion rate upon 1 day treatment with NH125.

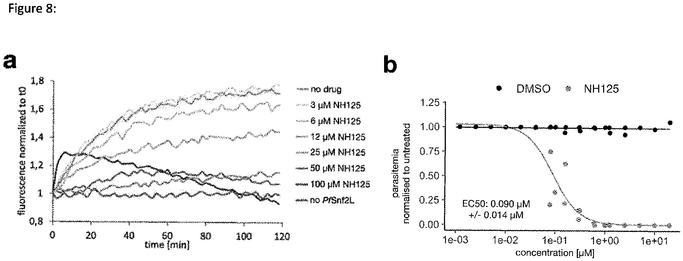

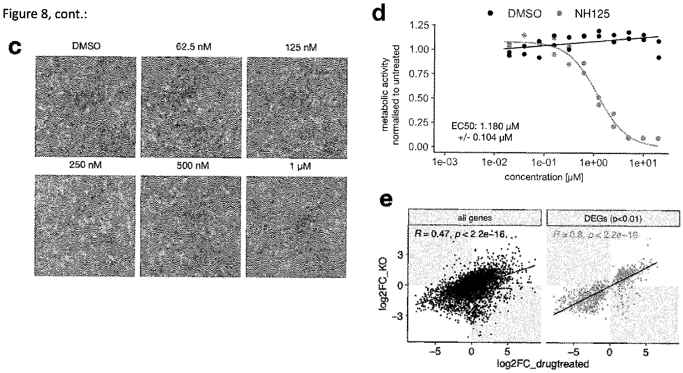

**Figure 8****: shows supplements to drug characterization, a,** ATP hydrolysis kinetic of *Pf*Snf2L in presence of NH125 in varying concentrations (grey) with normalized fluorescence of ParM as readout. *Pf*Snf2L (+DMSO) in absence of drug (green) serves positive control, samples without ATPase (red) as negative control. **b,** *Pf* Toxicity assay with parasitemia after 72 hours in presence of varying concentration of NH125 or DMSO correspondingly of three independent experiments, normalized to untreated parasitemia, EC50 determination by fitting dose response model. **c,** Toxoplasma toxicity. Exemplary plaque assay of Toxoplasma gondii infected human foreskin fibroblasts incubated for 1 week before fixation and staining **d,** HeLa toxicity. Metabolic activity of HeLa cells (monitored by XTT assay) in presence of varying concentration of NH125 or DMSO correspondingly of two independent experiments, normalized to untreated, EC50 determination as in B **e,** Pearson correlation between gene expression upon late *Pf*Snf2L-KO (+60) and upon NH125 treatment (+35) of all genes (left)/genes showing a significant change over both timecourse experiments (p<0.01) (right).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Role of the CRE Snf2L of the Plasmodium species P. falciparum

The inventors explored the functional role of the Plasmodium *Pf*Snf2L (PF3D7_1104200), which is most related to the ATP-dependent ISWI subfamily of CREs, but exhibiting only 30% homology to the human Snf2L (Fig. 1a,b). The protein contains a relatively conserved ATPase core but lacks conservation in the auto-regulatory and substrate-binding domains (Fig. 2a). The missing domains and large asparagine-rich insertions question its suggested activity as a nucleosome remodeling machine. Using the recombinantly purified *Pf*Snf2L, the inventors find that it specifically binds to nucleosomes (Fig. 3a-e) and the inventors show for the first time that this non-conserved enzyme moves histone octamers along DNA in an ATP-dependent manner (Fig. 2b). *Pf*Snf2L is a nucleosome-stimulated ATPase like ISWI enzymes, with an increased hydrolysis rate and reduced remodeling efficacy (Fig. 2c and Fig. 3f), potentially due to missing substrate recognition domains. Furthermore, the enzyme can re-assemble octamers from a DNA-histone mixture into well-positioned and functional nucleosomes in the absence of ATP (Fig. 2d).

Endogenous C-terminal tagging of *Pf*Snf2L revealed its expression in all asexual blood stages of 3D7 parasites, peaking in the late ring/trophozoite stage. Immunofluorescence analysis (IFA) showed its nuclear localization, being unevenly distributed in the nucleoplasm with spots of local enrichment (Fig. 2e). *Pf*Snf2L-immunoprecipitation combined with LC-MS/MS analysis identified specific interactors with a strong link to chromatin organization. The inventors detected 21 Apicomplexan- or Plasmodium-specific proteins, among them are several uncharacterized proteins. Neither the IP experiment nor *in silico* attempts identified orthologues of characteristic subunits of ISWI-type complexes in *Pf,* pointing towards the existence of novel divergent CRE complexes. Some interactors are also associated with transcription regulation and chromatin assembly, remodeling, and organization (Fig. 2f). For example, the FACT-complex plays an important role in nucleosome assembly during transcription elongation, while CAF- and NAP-proteins function in replication-coupled assembly. Other likely interactors are one or more versions of the GCN5 complex, as multiple GCN5 complex subunits were detected in the *Pf*Snf2LIP and the interaction was confirmed in a GCN5-IP. The results reveal a role for *Pf*Snf2L in nucleosome assembly and the chromatin-dependent regulation of gene expression.

To explore the biological function of *Pf*Snf2L in parasites, the inventors used an inducible dimerizable Cre-lox (DiCre)-mediated gene excision system⁽¹⁾. Efficient induction of gene excision (> 95 % induction rate) and resulting depletion of *Pf*Snf2L was verified at the DNA, mRNA and protein level 46 hours after addition of rapamycin. To analyze the phenotypic consequences of *Pf*Snf2L depletion, the inventors induced synchronized parasites directly (0 h) and 24 h post invasion (hpi). In both cases, development and replication are impaired in the second cycle prior to parasite death (Fig. 4a,b). Interestingly, early induction (at h) resulted in defects of merozoite release during host cell exit, as shown by live imaging (video), resulting in a reduced rate of newly infected red blood cells (RBCs). Until egress, the numbers of nuclei, schizont segmentation, cytogenesis, and invasion organelle formation remained unaffected. Staining of the host cell during egress indicates modified RBC properties at this specific time point in KO-induced (iKO) compared with non-induced parasites (noKO). The developmental defect in the second cycle is similarly observable when KO was induced at 24 hpi, but shifted in time, correspondingly. Taken together, the inventors found that *Pf*Snf2L plays an essential role in parasite survival and in driving the development of asexual stages.

To determine whether the drastic developmental effects were caused by perturbation of the parasite chromatin landscape, the inventors used MNase digestion and sequencing of schizont/late ring chromatin +/- *Pf*Snf2L-KO (Fig. 4c). The MNase-Seq method allows detection of individual nucleosome positions and, in addition, the monitoring of changes in the general accessibility of chromatin. MNase digestion kinetics at the schizont stage shows no detectable effect in chromatin accessibility, with noKO and iKO having a similar time-dependent DNA length pattern. However, after invasion, in the late ring stage, the chromatin of iKO parasites is more nuclease-accessible than in noKO parasites, releasing mono-nucleosomal DNA with lower MNase activity (Fig. 4c). Therefore, the loss of PfSnf2L induces a global opening of chromatin at the ring stage.

To analyze the local chromatin structure and individual nucleosome positions, noKO and iKO chromatin - digested to a similar degree - were analyzed in schizont and ring stages. Only minor changes of the nucleosome maps could be observed in schizonts (<1 %). However, the inventors found substantial changes in nucleosome positioning in late rings, where about 20 % of the well-positioned nucleosome were transformed. Changes in occupancy, fuzziness, and positioning are enriched in the 5'UTR and intergenic regions (Fig. 4e). These regions are associated with regulatory processes, comprising promoters with a highly defined chromatin architecture.

Drastic changes of the chromatin structure at regulatory elements upon *Pf*Snf2L-KO suggest effects on gene expression that may lead to the observed developmental defects. Time-resolved transcriptomic analysis of KO-parasites revealed a delay in gene expression timing (experimental timing in Fig. 5a). Genes being activated at specific stages are delayed in activation in the KO, and genes turning off also exhibit a later onset of repression (Fig. 5b). These changes in timing fidelity globally slowed down the circular trajectory of gene expression in the erythrocyte life cycle of the parasites, with the first effects visible approx. 40 h post KO induction (Fig. 6a and Fig. 5c). In total, 75 % of the genes are differentially expressed (DEG) at least one time point, with about 200 DEGs affected in schizont stage and approx. 2000 genes in ring and trophozoite stages (Fig. 6b). The large numbers as well as the enriched GO Terms show that *Pf*Snf2L-KO does not affect expression of specific gene categories. It rather shows that a loss of *Pf*Snf2L impacts those genes undergoing activation or repression in the respective developmental stage of the parasites. *Pf*Snf2L is likely required to maintain the timing of regulation of the entire parasite transcriptome. The gene expression changes are consistent with the phenotype of delayed cell cycle progression. In addition, the observed egress defect can be explained by the numerous exported proteins being downregulated in schizont stage. The collective knockdown of the multigene exported protein family (EPF) was previously shown to result in inefficient merozoite release, similar to the iKO of *Pf*Snf2L.

Interestingly, genes expressed outside of the asexual blood stages are upregulated in early rings, where they should be widely silenced (Fig. 6c). Among the mis-activated genes are numerous gametocyte-specific genes, which are mostly downregulated in trophozoite stage at the onset of sexual commitment, indicating that *Pf*Snf2L plays a role in the initiation of gametocytogenesis (Fig. 5d). This finding is supported by previous transcriptomic studies of gametocytogenesis, which identified elevated expression levels of *Pf*Snf2L in committing parasites. Having shown that known epigenetic regulators of sexual commitment, such as GDV1, are dysregulated by the lack of *Pf*Snf2L (Fig. 5e), the inventors propose this CRE to be a superordinate regulator of this regulatory network. In summary, the inventors find that *Pf*Snf2L is a key organizer of the nucleosomal landscape at gene promoters and its activity is decisive for just-in-time transcription activation/repression throughout the asexual and sexual intraerythrocytic stages of *Plasmodium falciparum* development.

Gene expression regulation occurs on multiple levels including transcriptional control, splicing, and RNA degradation, with the promoter being a major determinant. Its chromatin architecture, with positioned nucleosomes around a nucleosome-free region (NFR), controls the accessibility for transcription initiation factors and transcriptional activity. As the inventors observed the largest KO-dependent changes in chromatin structure in 5'UTRs and intergenic regions, the inventors examined nucleosome position changes at gene regulatory elements. Due to different published annotations of transcription start sites (TSS), the inventors used an alternative chromatin-based annotation method. The NucDyn pipeline was applied to identify the characteristic +1 nucleosome of promoters next to the proposed TSS. The alignment of MNase-Seq data relative to the well-positioned +1 nucleosome reveals the biggest changes in chromatin structure immediately upstream of +1. Subdivision of the data into gene expression quantiles shows the most pronounced effect of *Pf*Snf2L-KO on highly expressed genes (1^{st} quantile), resulting in a loss of nucleosome positioning (Fig. 6d, left panel). Furthermore, nucleosome-free regions tend to be extended and more accessible with increased expression in the noKO samples, but not in iKO, suggesting that *Pf*Snf2L actively opens the NFRs (mid panel). In agreement with these findings, the *Pf*Snf2L binding pattern at promoters changes with increasing gene expression levels, showing higher *Pf*Snf2L occupancy surrounding the NFR (right panel).

Highly expressed genes show the biggest KO-dependent changes in promoter architecture, and these genes also have the biggest KO-dependent changes in mRNA levels. In particular, genes that undergo strong activation or repression during the cycle - notably clonally variant genes - are *Pf*Snf2L-responsive (Fig. 6c,e). Upregulation in *Pf*Snf2L-KO coincides with a loss in nucleosome positioning at promoter regions, shown by increased nucleosome fuzziness (Fig. 6e). The opposite effect is observed for downregulated genes (Fig. 6e). The inventors propose that *Pf*Snf2L coordinates promoter architecture with gene expression timing. Supporting these results, ISWI-type CREs in other organisms have been shown to facilitate transcription initiation by enhancing the accessibility of trans-acting factors to gene promoters. Furthermore, the inventors found a direct interaction of *Pf*Snf2L with transcription factors and the Gcn5-complex, linking the processes of epigenetic and transcription regulation to *Pf*Snf2L function. In support, deletion of the GCN5/PHD1 domain has been shown to result in a delay in developmental progression, with reduced cycle transition and upregulation of non-blood stage-specific genes as seen for *Pf*Snf2L-KO.

### Example 2: Inhibition of the CRE Snf2L of the Plasmodium species P. falciparum

The essential role of *Pf*Snf2L in the malaria parasite, combined with its high sequence divergence from the human enzymes suggests that the protein is a potential drug target. Based on its enzymatic activity, a small molecule library was screened for inhibition and binding of the recombinant ATPase domain. The screening pipeline (Fig. 7a) combined an ATPase activity screen, followed by biophysical analysis of protein-small molecule interactions and *in vitro* nucleosome remodeling assays (Fig. 8a). Effective inhibitors were validated *in vivo,* and toxicity and specificity of the compounds were assessed. The anti-plasmodial chemical compound with the most pronounced effect was NH125 (Fig. 8a-d and Fig. 7b). Exposure of parasites to this compound mimics the *Pf*Snf2L-KO phenotype, exhibiting a similar developmental delay and parasite death as shown in Giemsa staining (Fig. 7c). Furthermore, time-resolved transcriptomic analysis after treatment with 1 µM of NH125 shows a highly significant correlation of DEGs when compared to the KO (Fig. 7d,e and Fig. 8e), confirming the direct and specific targeting of *Pf*Snf2L. Strikingly, the gametocyte conversion rate was dramatically decreased upon drug-treatment, emphasizing again the proposed function of *Pf*Snf2L in sexual commitment (Fig. 7f).

The inventors identified an essential role for *Pf*Snf2L in parasite viability, driving the developmental progress by controlling the timing of the transcriptional program. The protein is required for just-in-time activation and repression of stage-specific genes, and early regulation of gametocyte commitment. With that, the inventors could show the importance of epigenetic enzymes - in particular chromatin remodeling enzymes - for gene expression regulation in *Plasmodium falciparum.* CREs may compensate for the lack of transcription factors, being fine regulators of transcription timing and may further increase the regulatory potential by directly interacting with them. The CRE-transcription factor interplay may regulate the spatiotemporal loading of trans-acting factors on target genes and determine gene activity. Vice versa, it is possible that these interactions tune the enzymatic activity and nucleosome positioning behavior of *Pf*Snf2L.

*Pf*Snf2L lacks sequence conservation with other eukaryotic orthologues and additionally exhibits a distinct domain architecture. Remarkably, *Pf*Snf2L is an ATP-dependent nucleosome remodeling enzyme, altering nucleosome positions in vitro. Furthermore, the Plasmodium falciparum genome lacks any described chromatin remodeling complex subunits, which lead to the identification of a novel set of chromatin-related interactors. The functional divergence and its dissimilar interaction network suggest a specialized mode of action for Plasmodium CREs. These differences in comparison to other eukaryotes may have co-evolved with the structural and functional dissimilarities of the Plasmodium chromatin. *Pf*Snf2L may have been functionally adapted to a genome with high AT-content and rather unstable nucleosomes.

Consistent with the *Pf*Snf2L in vitro remodeling activity, knockout parasites reveal strong changes in the chromatin architecture, arguing that *Pf*Snf2L shapes the promoters of stage-specific genes and controls their transcriptional output. Its essential role and sequence divergence from other eukaryotic CREs, make it a potential drug target. Inhibiting its activity dramatically interferes with the intraerythrocytic cycle progression and gametocytogenesis, resulting in parasite death. The identified inhibitor with its high *Pf*Snf2L -specificity and pharmacological potency represents a tool to study the role of chromatin dynamics in Plasmodium development and is a solid basis for the further development into an antimalarial drug.

To conclude, the inventors identified *Pf*Snf2L (PF3D7_1104200) as ISWI-related ATPase actively repositioning *Pf* nucleosomes in vitro. Knockout studies demonstrate that *Pf*Snf2L is essential, regulating asexual development and sexual differentiation. It globally controls just-in-time transcription by spatiotemporally determining nucleosome positioning at promoters of stage-specific genes. The unique sequence and functional properties of *Pf*Snf2L allows the identification of an inhibitor that specifically kills *Plasmodium falciparum*, phenocopies the loss of correct gene expression timing, and inhibits the formation of gametocytes (NH125).

### Example 3: Comparison of ATPase domains of different Plasmodium species

The similarity of the ATPase domain of pfSnf2L of P. falciparum and the ATPase domain of Snf2L enyzmes of other species is shown in Table 4.

**Table 4: Comparison of ATPase domains of different Plasmodium species.**

| **Gene ID** | **Transcript ID** | **Species** | **Score** | **E-value** | **% identitiy** |
|---|---|---|---|---|---|
| PF3D7_1104200 | PF3D7_1104200.1 | *Plasmodium falciparum 3D7* | 1231 | 0E0 | 100 |
| PPRFG01_1104900 | PPRFG01_1104900_t1 | *Plasmodium praefalciparum strain G01* | 1231 | 0E0 | 100 |
| PRCDC_1102700 | PRCDC_1102700.1 *reichenowi* | *Plasmodium CDC* | 1226 | 0E0 | 99 |
| PGSY75_1104200 | PGSY75_1104200-t31_1 | *Plasmodium gaboni strain SY75* | 1225 | 0E0 | 99 |
| PVP01_0905000 | PVP01_{_}0905000.1 | *Plasmodium vivax P01* | 1139 | 0E0 | 92 |
| PmUG01_09013900 | PmUG01_09013900.1 | *Plasmodium malariae UG01* | 1137 | 0E0 | 92 |
| PcyM_0906000 | PcyM_0906000-t36_1 *cynomolgi* | *Plasmodium strain M* | 1120 | 0E0 | 91 |
| C922_04013 | C922_04013-t30_1 *Antonio* | *Plasmodium inui San 1* | 1118 | 0E0 | 92 |
| PRELSG_0901400 | PRELSG_09014900.1 | *Plasmodium relictum SGS1-like* | 1118 | 0E0 | 92 |
| PocGH01_09011000 | PocGH01_09011000.1 | *Plasmodium ovale curtisi GH01* | 1117 | 0E0 | 92 |
| AK88_03534 | AK88_03534-t30_1 | *Plasmodium fragile strain nilgiri* | 1116 | 0E0 | 91 |
| PGAL8A_00331500 | PGAL8A_00331500.1 *gallinaceum* | *Plasmodium 8A* | 1115 | 0E0 | 91 |
| PVVCY_0900350 | PVVCY_0900350_t1 | *Plasmodium vinckei vinckei CY* | 1111 | 0E0 | 92 |
| PKNH_0901800 | PKNH_0901800.1 | *Plasmodium knowlesi strain H* | 1109 | 0E0 | 91 |
| PY17X_0945200 | PY17X_0945200.1 | *Plasmodium yoelii yoelii 17X* | 1105 | 0E0 | 91 |
| PBANKA_0942700 | PBANKA_0942700.1 | *Plasmodium berghei ANKA* | 1093 | 0E0 | 91 |

| | | | | | |
|---|---|---|---|---|---|
| Of note, a compound targeting the ATPase domain of pfSnf2L is also highly likely to also target a similar ATPase domain of a Snf2L enyzme of other species, such as any one listed in Table 4. | | | | | |

### Example 4: Material and Methods:

### • Phylogenetic analysis and multiple sequence alignments

Sequences of CREs used for phylogenetic analysis are listed in Table 5:

| Fig. | name in tree | organism | UniProt ID | used for phylogenetic analysis (aa) | | |
|---|---|---|---|---|---|---|
| 1A | dmAtrx | Drosophila melanogaster | Q9GQN5 | 455-782 | 897-1070 | |
| 1A | dmChd1 | Drosophila melanogaster | Q7KU24 | 528-811 | 835-987 | |
| 1A | dmEtl1 | Drosophila melanogaster | Q9VL72 | 289-586 | 651-802 | |
| 1A | dmIno80 | Drosophila melanogaster | Q9VDY1 | 525-839 | 1155-1306 | |
| 1A | dmISWI | Drosophila melanogaster | Q24368 | 128-410 | 430-582 | |
| 1A | dmLodestar | Drosophila melanogaster | P34739 | 439-486 | 524-804 | 889-1041 |
| 1A | dmMi2 | Drosophila melanogaster | 097159 | 730-1026 | 1051-1203 | |
| 1A | dmMot1 | Drosophila melanogaster | Q9VF02 | 1350-1661 | 1689-1850 | |
| 1A | dmRad54 | Drosophila melanogaster | O76460 | 156-464 | 497-649 | |
| 1A | dmShprh | Drosophila melanogaster | Q9VRV2 | 160-247 | 334-1251 | |
| 1A | dmSmarcal1 | Drosophila melanogaster | Q9VMX6 | 247-755 | | |
| 1A | dmSnf2 | Drosophila melanogaster | P25439 | 773-1070 | 1079-1249 | |
| 1A | dmSwr1 | Drosophila melanogaster | Q9NDJ2 | 914-1202 | 1657-1808 | |
| 1A | hsAlc1 | Homo sapiens | Q86WJ1 | 46-327 | 346-496 | |
| 1A | hsArip4 | Homo sapiens | Q9Y4B4 | 263-628 | 720-889 | |
| 1A | hsAtrx | Homo sapiens | P46100 | 1560-1889 | 2017-2190 | |
| 1A | hsChd1 | Homo sapiens | O14646 | 481-764 | 787-939 | |
| 1A | hsChd7 | Homo sapiens | Q9HCK8 | 811-1100 | 1132-1284 | |
| 1A | hsEP400 | Homo sapiens | Q96L91 | 1091-1373 | 1897-2048 | |
| 1A | hsErcc6 | Homo sapiens | Q03468 | 507-811 | 838-987 | |
| 1A | hsEtl1 | Homo sapiens | Q9H4L7 | 497-787 | 853-1004 | |
| 1A | hsIno8o | Homo sapiens | Q9ULG1 | 518-821 | 1100-1251 | |
| 1A | hsISWI_1 | Homo sapiens | P28370 | 183-649 | | |
| 1A | hsLodestar | Homo sapiens | Q9UNY4 | 570-617 | 641-932 | 990-1141 |
| 1A | hsLsh | Homo sapiens | Q9NRZ9 | 223-576 | 598-749 | |
| 1A | hsMi2 | Homo sapiens | Q12873 | 736-1034 | 1059-1211 | |
| 1A | hsMot1 | Homo sapiens | O14981 | 1266-1584 | 1613-1781 | |
| 1A | hsRads | Homo sapiens | Q14527 | 240-315 | 477-721 | 832-987 |
| 1A | hsRad54 | Homo sapiens | Q92698 | 153-461 | 494-646 | |
| 1A | hsShprh | Homo sapiens | Q149N8 | 304-394 | 715-1661 | |
| 1A | hsSmarcal1 | Homo sapiens | Q9NZC9 | 436-954 | | |
| 1A | hsSnf2 | Homo sapiens | P51532 | 754-1051 | 1079-1231 | |
| 1A | hsSwr1 | Homo sapiens | Q6ZRS2 | 618-906 | 2042-2193 | |
| 1A | scChd1 | Saccharomyces cerevisiae | P32657 | 376-664 | 694-846 | |
| | | Saccharomyces | | | | |
| 1A | scErcc6 | cerevisiae | P40352 | 297-632 | 650-803 | |
| | | Saccharomyces | | | | |
| 1A | scEtl1 | cerevisiae | P31380 | 572-849 | 948-1100 | |
| | | Saccharomyces | | | | |
| 1A | scIn080 | cerevisiae | P53115 | 706-1016 | 1298-1449 | |
| | | Saccharomyces | | | | |
| 1A | scISWI_1 | cerevisiae | P38144 | 196-481 | 501-653 | |
| | | Saccharomyces | | | | |
| 1A | scLsh | cerevisiae | P43610 | 222-754 | | |
| | | Saccharomyces | | | | |
| 1A | scMot1 | cerevisiae | P32333 | 1272-1579 | 1608-1783 | |
| | | Saccharomyces | | | | |
| 1A | scRad5 | cerevisiae | P32849 | 437-553 | 585-847 | 993-1152 |
| | | Saccharomyces | | | | |
| S1A | scRad16 | cerevisiae | P31244 | 184-226 | 230-772 | |
| | | Saccharomyces | | | | |
| 1A | scRad54 | cerevisiae | P32863 | 271-620 | 654-805 | |
| 1A | scRis1 | Saccharomyces cerevisiae | Q08562 | 943-990 | 996-1280 | 1442-1596 |
| 1A | scSnf_1 | Saccharomyces cerevisiae | P32597 | 470-942 | | |
| 1A | scSwr1 | Saccharomyces cerevisiae | Q05471 | 696-998 | 1245-1396 | |
| 1A | PF3D7_1343400 | Plasmodium falciparum | Q8IDQ5 | 661-705 | 742-1452 | |
| 1A | PF3D7_1104200 | Plasmodium falciparum | Q8IIW0 | 322-777 | | |
| 1A | PF3D7_1023900 | Plasmodium falciparum | Q8IJG6 | 1296-1764 | | |
| 1A | PF3D7_080340 | Plasmodium falciparum | Q8IAN4 | 195-1239 | | |
| 1A | PF3D7_062460 | Plasmodium falciparum | C6KT82 | 338-673 | 1084-1228 | |
| 1A | PF3D7_0106000 | Plasmodium falciparum | B9ZSI3 | 1-922 | | |
| 1A | PF3D7_0216000 | Plasmodium falciparum | O96239 | 884-1485 | | |
| 1A | PF3D7_0818700 | Plasmodium falciparum | CoH4V8 | 208-1223 | | |
| 1A | PF3D7_082000 | Plasmodium falciparum | CoH4W3 | 662-951 | 1767-1918 | |
| 1A | PF3D7_1357500 | Plasmodium falciparum | Q8IDD0 | 353-795 | | |
| 1B | atISWI | Arabidopsis thaliana | F4JY24 | full length | | |
| 1B | cmISWI | Cyanidioschyzon merolae | M1VKV9 | full length | | |
| 1B | cpISWI | Cryptosporidiu m parvum | Q5CVU2 | full length | | |
| 1B | dmISWI | Drosophila melanogaster | Q24368 | full length | | |
| 1B | hsISWI_1 | Homo sapiens | P28370 | full length | | |
| 1B | hsISWI_2 | Homo sapiens | O60264 | full length | | |
| 1B | mmISWI_1 | Mus musculus | Q91ZW3 | full length | | |
| 1B | mmISWI_2 | Mus musculus | Q6PGB8 | full length | | |
| 1B | ncISWI | Neospora caninum | F0VIN7 | full length | | |
| | | Plasmodium | A0A509AKD | | | |
| 1B | pbISWI | bergheii | 2 | full length | | |
| 1B | ptISWI | Phaeodactylum tricornutum | B7G2T7 | full length | | |
| 1B | scISWI_1 | Saccharomyces cerevisiae | P38144 | full length | | |
| 1B | scISWI_2 | Saccharomyces cerevisiae | Q08773 | full length | | |
| 1B | tcISWI | Trypanosoma cruzii | Q4CNC6 | full length | | |
| 1B | tgISWI | Toxoplasma gondii | S7V4B2 | full length | | |
| 1B | xlISWI | Xenopus laevis | Q6DFM0 | full length | | |
| 1B | PF3D7_1104200 | Plasmodium falciparum | Q8IIW0 | full length | | |

Phylogenetic trees were generated using *Geneious Tree Builder* (Geneious software 11.1.5): Global alignment with free end gaps, Blosum62 as Cost Matrix, Jukes-Cantor Distance Model, Method Neighbor-Joining, Gap open penalty 10 and Gap extension penalty 0.2. For multiple sequence alignments (MSA), *Geneious Alignment* with same parameter, but Gap open penalty 20 and 5 refinement iterations was used. Pairwise identity within and between Metazoan/Apicomplexan proteins was calculated from MSA and kernel smoothing was applied.

### • Nucleosome reconstitution

DNA templates for nucleosome assembly were synthesized by PCR, using (Cy3-/Cy5-labelled) oligonucleotides binding flanking regions of 147 bp nucleosome positioning sequence (NPS) creating various linker lengths: 0-NPS-0, 6-NPS-47, 77-NPS-77. Recombinant histones - canonical *Hs*/*Pf* and variant *Pf* (H₂A.Z/B.Z containing) - were expressed, purified and refolded as previously described. *Bt* and *Gg* octamers were purified from calf thymus, or chicken blood respectively (Fig. 3c). Nucleosome assembly was performed via gradient dialysis: Suitable ratios of histone octamer: DNA were mixed in 40-200 µl high salt buffer (10 mM Tris pH 7.6/2 M NaCl/1 mM EDTA/1 mM 2-mercaptoethanol/0.05% Igepal CA-630) supplemented with 200 ng/µl BSA and placed into small dialysis chambers. Starting in a beaker with 300 ml high salt buffer, salt concentration was slowly reduced by pumping 3 l of low salt buffer (10 mM Tris pH 7.6/50 mM NaCl/1 mM EDTA/1 mM 2-mercaptoethanol/0.05% Igepal CA-630) to it. Reconstituted nucleosomes were analyzed by gel electrophoresis, using 6% PAA 0.4xTBE gels and visualized by fluorescence scanning (Typhoon FLA-9500) or ethidium bromide staining (Fig. 3d).

### • CRE expression and purification

*Pf*Snf2L (aa250-1426) was sequence-optimized with addition of a C-terminal 10xHis-Tag, ordered as synthetic gene (IDT), and cloned into pFL plasmid. After transformation in DH10Bac EM YFP cells, bacmid DNA was isolated and transfected into *Spodoptera frugiperda* Sf21 cells (Invitrogen). The produced initial virus was further propagated and used for large scale expression. Cells expressing recombinant *Pf*Snf2L-10xHis or *Hs*Snf2L-6xHis were harvested and lysed in 20 mM Tris-HCl pH 7.6/500 mM KCl/1.5 mM MgCl2/0.5 mM EGTA/5 mM β-mercaptoethanol/10% glycerol/0.1% Igepal CA-630 using Branson Sonifier 250. Purification was performed via NiNTA agarose (Qiagen) according to manufacturer's recommendation. Protein concentration was estimated via Bradford assay and the purity was checked with Coomassiestained SDS-PAGE.

### • Nucleosome binding, assembly, remodeling assays

*In vitro* reconstituted nucleosomes in 10 µl 20 mM Tris pH 7.6/100 mM KCl/1.5 mM MgCl₂/0.5 mM EGTA/10% glycerol/200 ng/µl BSA were incubated for 60 min at 30°C in absence/presence of recombinantly expressed CRE. In competitive binding assays 2× 15 nM nucleosomes + 0.02-0.6 µM CRE, in non-labelled binding assays and assembly assays 120 nM nucleosome + 700 nM CRE, in remodeling assays 30 nM nucleosome + 300 nM CRE + 1 mM ATP was used. Assembly and remodeling reactions were stopped after 60 min (unless noted differently) by addition of 1 µg competitor plasmid DNA and nucleosome positions were analyzed via 6 % native PAGE.

### • ATPase assays

CRE (130-700 nM) in 20 mM Tris-HCl pH 7.6/120 mM KCl/1.5 mM MgCl2/0.5 mM EGTA/10% glycerol was incubated together with 500 µM ATP and 0.2 µCi ³²P-γ-ATP in absence or presence of mononucleosomes (60-350 nM, various compositions) for 40 min at 30°C. Released ³²P-γ phosphate was separated from non-hydrolyzed ³²P-γ-ATP in three technical replicates by TLC on PEI-Cellulose F plates (Merck) [mobile phase: 50% acetic acid, 0.5 mM LiCl]. After phosphoimaging (Typhoon FLA-9500), signal intensities were quantified (Fuji Multi Gauge Software), the hydrolysis rate was calculated, corrected for chemical hydrolysis, and normalized to CRE concentration.

### • Plasmid construction

A synthetic gene comprising of the native nucleotides 995-1894 of PF3D7_1104200, an artificial intron with loxP site, nucleotides 1895-4278 recodonized and a 3×HA-tag encoding sequence was ordered as synthetic gene (IDT) and cloned into the vector pT2A-X-KO. The resulting plasmid pT2A-Snf2L-KO contains downstream of the P*f*Snf2L-encoding part a skip peptide, a neomycin resistance gene to enable selection-linked integration, a second loxPsite and a GFP gene as well as an independent hDHFR gene.

### • Parasite culture & transfection

*P. falciparum* clone 3D7 was cultured according to standard procedures in RPMI 1640 with Albumax^{®} (Invitrogen), and synchronized adapted from 54: Schizonts were purified on a bed of 70% Percoll^{®}, incubated with new red blood cells for 1-2 h, before leftover schizonts were removed with Percoll and subsequent sorbitol treatment. About 10 µg of plasmid was used for transfection of DiCre-expressing parasites with the Amaxa^{™} P₃ primary cell 4D-Nucleofector^{™} X Kit L (Lonza, Cologne, Germany). Successful transfection was selected with 2.5 nM of the antifolate WR99210 (Jacobus Pharmaceutical Company, Princeton, NJ, USA), starting one day post transfection with frequent exchange of media. Once resistant parasites were established, they were selected for genomic integration with 400 ug/ml G418 (Sigma Aldrich). After limiting dilution of drug-resistant parasites, genomic DNA of six clones was isolated using Qiagen Blood and Tissue kit. Integration was confirmed via genotyping PCR using Q5 polymerase (NEB), and one clone was used for further phenotyping. Conditional DiCre-mediated recombination between loxP sites was performed as described ⁽¹⁾. For KO induction, synchronous parasites were treated with 100 nM rapamycin (Sigma Aldrich) for 4 h (DMSO treatment as control), washed and returned to culture. Gene excision was confirmed by genotyping PCR, mRNA levels were checked in RNAseq samples, protein level via Western blot.

### • Western blot analysis

Parasites were isolated from RBCs by 0.1% saponin and protease inhibitor cocktail PIC (Roche) in PBS and boiled for 10 min in 62 mM Tris pH6.5, 25 % glycerol, 2 % SDS, 0.2 M DTT, 0.05 % OrangeG. Proteins were separated on 4-20 % SDS gels (Biorad), transferred onto a nitrocellulose membrane prior to immunoblotting. In this study, rat anti-HA (1:2000, Roche) and mouse anti-Enolase (1:1000, Prof GK Jarori, Tata Institute for Fundamental Research) were used, conjugated secondary antibodies and Odyssey^{®} imaging system (LiCOR Biosciences) according to manufacturer's recommendation.

### • Immunofluorescence assays (IFA)

Immunofluorescence assay were performed as in with the addition of 0.0075 % glutaraldehyde during fixation. Primary antibodies (rat anti-HA, rabbit anti-Gap45, rabbit anti-Ama1) were used 1:500. For image acquisition, Leica DMi8 Widefield microscope was used, image processing and quantification was performed with software Fiji v2.9.0 (ImageJ).

### • Analysis of parasite growth (PG), reproduction (PR) and egress (PE)

Growth was determined by microscopic counting of parasites from Giemsa-stained thin blood films and expressed as percentage parasitemia (% infected RBCs/RBCs). For reproduction and egress assays, equal numbers of mature schizonts were Percoll-purified. For PR, they were incubated in 4 ml RPMI with 1 % hematocrit for 2 h, before the number of rings was quantified from Giemsa stain. Live imaging of PE was performed as described 55, parasites were binned in groups 15 sec post egress (normal / clustered merozoites). For RBC staining, RPMI was supplemented with 0.1x phalloidin 594 Conjugate (Abcam, Cambridge) during live imaging. RBC ghost location 40 sec post egress was categorized (distant/attached/overlapping).

### • Transmission Electron Microscopy (TEM)

Schizont stage parasites (46 hpi) were treated with compound2 for 2 h for further maturation. Then they were washed in PHEM buffer (2.5 mM MgCl₂/35 mM KCl/₅ mM EGTA/10 mM Hepes/30 mM PIPES, pH 7.2), fixed for 1 hour in a solution containing 2.5 % glutaraldehyde, 4% formaldehyde, 4% sucrose in 0.1 M PHEM buffer, postfixed in 1 % OsO₄ plus 0.8 % ferrocyanide and 5 mM CaCl₂ in 0.1 M cacodylate buffer for 1 hour, washed twice in cacodylate buffer (10 min, 1 h) and double-distilled water (5 min, 15 min), respectively and dehydrated in a graded acetone series which included *en bloc* staining with 1 % uranyl acetate in the 20 % step. The cells were finally embedded in Epon812 epoxy resin to enable ultrathin sectioning. To carry out electron microscopy, the ultrathin sections were post-stained with 1 % lead citrate for 2 min. Transmission electron microscopy was carried out using either a Zeiss EM 912 (Zeiss AG, Oberkochen, Germany), set to an acceleration voltage of 80 kV, operated in the zero-loss mode and equipped with a 2k × 2k slow-scan CCD camera (TRS Tröndle Restlichtverstärkersysteme, Moorenweis, Germany) or a JEOL F200 (JEOL, Freising, Germany), operated at 200 kV equipped with a XAROSA 20 mega pixel CMOS camera (EMSIS, Münster, Germany.)

### • Preparation of nuclear extracts and Co-immunoprecipitation (co-IP) experiments

Nuclei of P*f*Snf2L-HA and 3D7 parasites were prepared as in and treated with 0.5 U/µl benzonase (Sigma Aldrich) in 20 mM HEPES pH7.9/10 mM KCl/0.1 mM EDTA/0.1 mM EGTA/1 mM DTT/0.65 % Igepal CA-630 and PIC for 30 min at room temperature. For extraction of nuclear proteins, KCl was added to 0.4 M, incubated for 30 min at room temperature and insoluble fraction was removed by centrifugation (5000 g, 30 min, 4 °C). The supernatant was diluted with 2.5 vol of 50 mM Tris pH 7.4/50 mM NaCl/1 mM EDTA/1 % Igepal CA-630 and PIC, and incubated overnight at 4°C under constant agitation with equilibrated anti-HA magnetic beads (Thermo Fisher). Beads were pelleted using a magnetic rack, washed 3x with latter buffer and 3x with 50 mM Tris-HCl pH 8. Beads were dried, stored at -20 °C and used for LC-MS/MS analysis of co-immunoprecipitated proteins in P*f*Snf2L-HA parasites (3D7 as negative control), in triplicates each. Collected fractions (input, flowthrough, beads) were analyzed on 4-20 % SDS-PAGE, silver-stained and in additional version probed anti-HA in western blot.

### • Mass spectrometry (LC-MS/MS) and data analysis

Beads were incubated with 10 ng/µL trypsin in 1 M urea/50 mM NH₄HCO₃ for 30 min, washed with 50 mM NH₄HCO₃, and the supernatant digested overnight in presence of 1 mM DTT. Digested peptides were alkylated and desalted prior to LC-MS analysis. For LC-MS/MS purposes, desalted peptides were injected in an Ultimate 3000 RSLCnano system (Thermo Fisher), separated in a 15\-cm analytical column (75 µm ID with ReproSil-Pur C18-AQ 2.4 µm from Dr. Maisch) with a 50-min gradient from 4 % to 40 % acetonitrile in 0.1 % formic acid. The effluent from the HPLC was directly electrosprayed into a QexactiveHF (Thermo Fisher) operated in data dependent mode to automatically switch between full scan MS and MS/MS acquisition. Survey full scan MS spectra (from m/z 350 to 1,600) were acquired with resolution R = 60,000 at m/z 400 (AGC target of 3 × 10⁶). The 10 most intense peptide ions with charge states between 2 and 5 were sequentially isolated to a target value of 1 × 10⁵ and fragmented at 30 % normalized collision energy. Typical mass spectrometric conditions were as follows: spray voltage, 1.5 kV; heated capillary temperature, 275 °C; ion selection threshold, 33.000 counts.

MaxQuant 1.6.14.0 was used to identify proteins and quantify by iBAQ with the following parameters: Database Uniprot_UP000001450_Plasmodiumfalciparum_20201007.fasta; MS tol, 10 ppm; MS/MS tol, 20 ppm Da; PSM FDR, 0.01; Protein FDR, 0.01 min; Peptide Length, 7; Variable modifications, Oxidation (M), Acetyl (Protein N-term); Fixed modifications, Carbamidomethyl (C); Peptides for protein quantitation, razor and unique; Min. peptides, 1; Min. ratio count, 2. MaxQuant iBAQ values were log2 transformed, missing values implemented with 8. Ribosomal proteins and hits detected with only 1 peptide were excluded. Identified proteins were considered as interaction partners of the bait, if log₂(Snf2L-₃HA) - log₂(3D7) > 3 or log₂(Snf2L-3HA) - log₂(3D7) > 2 & FDR <0.05. The mass spectrometry proteomics data have been deposited to the ProteomeXchange Consortium via the PRIDE ⁶⁰ partner repository with the dataset identifier PXD041155.

### • RNA-seq and data analysis

RNA-sequencing +/- Snf2L-KO and +/- drug, respectively, was performed in triplicates. Total RNA from iRBCs containing highly synchronized parasites was isolated using Whole Blood Quick RNA kit (Zymo Research) according to the manufacturer's protocol. RNA quality was checked using 4200 TapeStation System (Agilent) and 300 ng were used as input for Illumina Stranded mRNA Prep Ligation (Illumina). Libraries were sequenced on the Illumina NextSeq 2000 Sequencing System. Sequenced reads (2×57bp bp, paired-end, ~20 Mio reads/sample) were trimmed with trimmomatic vo.39 and mapped to *P. falciparum* 3D7 genome v3.0 (plasmoDB.org, release 52) using STAR 2.7.9a. PlasmoDB annotation was converted to GTF-format with gffread 0.12.1.Preprocessing and mapping quality control was done with FastQC 0.11.8, qualimap 2.2.2d, samtools 1.12 and multiqc 1.9. The pipeline was implemented with snakemake 5.32.0. RSubread/FeatureCounts 2.12.2 was utilized to calculate read counts, while differential expression analysis was performed by DESeq2 adjusted P-value <0.05 as significance cutoff. Further analysis and visualization were done in R using tidyverse and ggpubr. DEGreport was used for clustering and topGO for GO term enrichment analysis. For expression quantile calculation transcript per millions (TPM) normalization was used. RNA-Seq data were submitted to Gene Expression Omnibus (GEO) database (accession number GSE228949). RNA-seq-based cell cycle progression was estimated in R by comparing the normalized expression values of each sample to the RNAseq data from using the statistical model previously described. P*f*Snf2L-knockout efficacy was estimated by mapping reads to the sequence of the recodonized transfected Snf2L gene and counting reads mapped to the Snf2L_{recodon} part of the gene which is disintegrated upon knockout induction.

### • MNase-seq + data analysis

MNase-digestion was adapted from⁽²⁾ with following modifications: Highly synchronous parasites were crosslinked and stopped as described. RBCs were lysed and nuclei were isolated as for pulldown experiments. Nuclei were resuspended in 75 µl 50 mM Tris pH7.4/4 mM MgC12/1 mM CaCl2/0.075% NP40/1 mM DTT, PIC with 0.75 U Micrococcal nuclease (Worthington Biochemicals Corporation) and 50 U Exonuclease III (NEB). Each sample was aliquoted in 3 × 25 µl and incubated for 2.5 / 5 / 10 min at 37 °C under agitation (low/mid/high digestion), before the reaction was stopped by adding 25 µl 2 % Triton X100/0.6 % SDS/300 mM NaCl/6 mM EDTA/PIC. and placed at 4 °C. Decrosslinking was performed at 45 °C overnight after adjusting to 1 % SDS/0.1 M NaHCO3/0.5 M NaCl. Proteins were digested by addition of 40 µg of Proteinase K (Zymo Research) and incubation for 1 h at 55 °C. Subsequently, DNA was isolated using EXTRACTME DNA clean-up kit and Micro Spin columns (Blirt) as the manufacturer recommends.

Digestion efficacy of extracted DNA fragments were analyzed with TapeStation 4200 System. Molarity-normailzed DNA size distribution was visualized using BioanalyzeR, kinetic is shown as concentration-weighted mean DNA length over time. For low digested samples, DNA fragments >700 bp were removed using SPRI beads, no size-selection step was applied for high digested samples. 1 ng of high/low digested samples was used as input for NEBNext Ultra II DNA library prep kit (NEB), according to manufacturer's protocol. Libraries were sequenced on the Illumina NextSeq 2000 Sequencing System. Sequenced reads (2×65 bp, paired-end, ~20 Mio reads/sample) were trimmed with skewer 0.2.2 and subsequently mapped to *P.falciparum* 3D7 genome v3.0 (plasmoDB.org, release 52) using Bowtie2 2.3.5 with "--very-sensitive --no-discordant --no-mixed --no-unal" options. Mapped fragments were filtered with deeptools 3.5.0 ⁸¹ for a mapping quality of at least 20, a fragment size between 75 -175 bp and proper read pairs. The remaining fragments were used for nucleosome calling with DANPOS (custom version, based on DANPOS3). Quality control was done with FastQC 0.11.8, qualimap 2.2.2d, samtools 1.12 and multiqc 1.9.The pipeline was implemented with snakemake 5.32.0, MNase-Seq data at GEO (GSE228949). Further analysis was done with R etc.

### • ADP biosensor assay

ATPase activity and inhibition were measured by ADP biosensor assay: In 20 µl, 0.2 µM TMR-maleimide-labelled ParM was mixed with 125 µM ATP, 100 ng plasmid pT11 DNA, 5 µM H4 peptide (aa8-25, AnaSpec) in 10 mM Tris pH8.6/1.5 mM MgCl₂/100 mM KCl/0.01 % Pluronic in presence (or absence as negative control) of 0.4 µM recombinantly expressed P*f*Snf2L and NH125 in varying concentrations. Resulted ADP binding to ParM is expressed by increasing fluorescence intensity and was kinetically measured at 28 °C in 2 min intervals over 2 h in a Tecan infinite F500 reader. Signal was normalized to timepoint 0.

### • Toxicity assay Plasmodium / Toxoplasma / HeLa

Toxicity for Plasmodium was determined by culturing in presence of NH125/DMSO for 72 hours and subsequent quantification of parasitemia in Giemsa-stained blood films. Toxicity for Toxoplasma was tested via plaque assay, whereby 1000 *T. gondii* tachyzoites inoculated onto human foreskin fibroblasts as in were treated with NH125/DMSO, fixed and stained 6 days later and evaluated with regard to plaque formation. Toxicity on human HeLa cells was investigated by 48 hour NH125/DMSO treatment and subsequent monitoring of metabolic activity using the Cell Proliferation Kit II-XTT (Sigma Aldrich) according to manufacturer's recommendation. EC50 values for A/C were obtained by fitting dose response model using three-parameter log-logistic models and estimated for **b,**

### • Gametocyte induction

Gametocytes were generated using the nutrient deprivation induction method as described Briefly, synchronized trophozoites were cultured at ~4-5 % parasitemia before gametocytogenesis was induced by splitting out the culture to 2 % parasitemia in minimal fatty acid (mFA) media (Day 0). With that, NH125 / DMSO treatment was started. 22 hours post induction, media was replaced back to complete RPMI media. On Day 2, parasitemia was quantified in Giemsa-stained blood smears and 20 U/ml Heparin was added to the culture. The media was changed daily until quantification of gametocytemia on Day 8. Gametocyte conversion rate was calculated: GCR=Gametocytemia-Day8/Parasitemia-Day2.

### REFERENCES

The references are:
1. Collins, C. R. et al. Robust inducible Cre recombinase activity in the human malaria parasite Plasmodium falciparum enables efficient gene deletion within a single asexual erythrocytic growth cycle: Conditional Cre in the human malaria parasite. Molecular Microbiology 88, 687-701 (2013).
2. Kensche, P. R. et al. The nucleosome landscape of Plasmodium falciparum reveals chromatin architecture and dynamics of regulatory sequences. Nucleic Acids Research 44, 2110-2124 (2016).

## Claims

1. A compound for use in a method of diagnosis, prevention, and/or treatment of a Malaria infection in a subject, wherein the compound is an inhibitor of a Chromatin remodeling enzyme (CRE) of a Plasmodium, or a related species of the Apicomplexa, Amoebozoa or Euglenozoa group, and wherein said CRE has a CRE ATPase domain.

2. The compound for use according to claim 1, wherein the compound alters the development and/or the life cycle of the Plasmodium or the related species of the Apicomplexa, Amoebozoa or Euglenozoa group, inhibits the growth of the Plasmodium or the related species of the Apicomplexa, Amoebozoa or Euglenozoa group, inhibits the survival of the Plasmodium or the related species of the Apicomplexa, Amoebozoa or Euglenozoa group, and/or kills the Plasmodium or the related species of the Apicomplexa, Amoebozoa or Euglenozoa group.

3. The compound for use according to claim 1 or 2, wherein the compound is an inhibitor of a CRE Snf2L of the Plasmodium or the related species of the Apicomplexa, Amoebozoa or Euglenozoa group, and wherein the compound binds to the CRE Snf2L of the Plasmodium or the related species of the Apicomplexa, Amoebozoa or Euglenozoa group, optionally wherein the CRE Snf2L repositions nucleosomes in an ATP-dependent manner, and/or moves histone octamers along a nucleic acid, such as a DNA, further optionally wherein the compound is binding to and/or is specifically inhibiting the CRE Snf2L in the Plasmodium or the related species of the Apicomplexa, Amoebozoa or Euglenozoa group.

4. The compound for use according to any one of claims 1 to 3, wherein the compound controls the gene expression in the Plasmodium or the related species of the Apicomplexa, Amoebozoa or Euglenozoa group via altering the positions of the nucleosomes and/or affecting the structure of chromatin, optionally wherein the compound spatiotemporally controls nucleosome positioning at promoters of stage-specific genes, and thereby controls just-in-time transcription, activation and/or repression of stage-specific genes, further optionally wherein the compound is an epigenetic modulator.

5. The compound for use according to any one of claims 1 to 4, wherein the compound represses gametocyte-specific gene expression, and thereby inhibits the formation of gametocytes of the Plasmodium or the related species of the Apicomplexa, Amoebozoa or Euglenozoa group, and/or prevents the initiation of gametocytogenesis in the Plasmodium or the related species of the Apicomplexa, Amoebozoa or Euglenozoa group.

6. The compound for use according to any one of claims 1 to 5, wherein the CRE ATPase domain has a sequence homology of no more than 40% to the amino acid sequence of full length human Snf2L according to SEQ ID No: 1, preferably of no more than 35%, more preferably of no more than 30%, and most preferably of around 26% sequence homology with respect to the full length human Snf2L sequence according to SEQ ID No: 1.

7. The compound for use according to any one of claims 1 to 6, wherein the CRE is selected from any one of the CRE Snf2L of the Plasmodium species P. falciparum, P. vivax, P. malariae, P. ovale, P. knowlesi, P. cynomolgi, P. berghei, P. praefalciparum, P. reichenowi, P. gaboni, P. inui San Antonio 1, P. relictum SGS1-like, P. fragile, P. gallinaceum, P. vinckei vinckei, P. yoelii, and their variants, preferably having an amino acid sequence according to any one of SEQ ID Nos: 2-17.

8. The compound for use according to any one of claims 1 to 7, wherein the compound is 1-Benzyl-3-cetyl-2-methylimidazolium iodide (NH125) according to Formula (I) 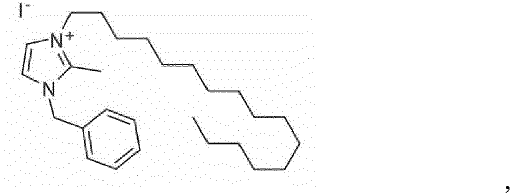 or a derivative thereof, wherein the derivate is a molecule with similar chemical properties compared to 1-Benzyl-3-cetyl-2-methylimidazolium iodide (NH125) according to Formula (I), and wherein the derivative thereof is preferably selected from:
Benzyl(hexadecyl)dimethylazanium (Cetalkonium), Benzyldimethyltetradecylazanium (Myristalkonium), Benzyl(dodecyl)dimethylazanium (Benzododecinium), Hexadecyl(2-{[(4-methoxyphenyl)methyl](pyrimidin-2-yl)amino}ethyl)dimethylazanium (Thonzonium), 1-hexadecylpyridin-1-ium (Cetylpyridinium), {10-[dimethyl(2-{[(1R,2S,5R)-5-methyl-2-(propan-2-yl)cyclohexyl]oxy}-2-oxoethyl)azaniumyl]decyl}dimethyl(2-{[(1R,2S,5R)-5-methyl-2-(propan-2-yl)cyclohexyl]oxy}-2-oxoethyl)azanium (Decamethoxine), Hexadecyl 2-ethylhexanoate (Cetyl ethylhexanoate), Methyl 2-[1-(2-{4-[(quinolin-2-yl)methoxy]phenyl}ethyl)piperidin-4-ylidene]-4,7-diazatricyclo[8.4.0.0^{3,7}]tetradeca-1(14),3,5,10,12-pentaene-6-carboxylate (Laniquidar), 1,3-bis(heptanoyloxy)propan-2-yl heptanoate (Triheptanoin), 2-{[(2R)-1-(4-{4-[3-(azepan-1-yl)propoxy]phenyl}butyl)pyrrolidin-2-yl]methyl}-4-[(4-chlorophenyl)methyl]-1,2-dihydrophthalazin-1-one (GSK-1004723), (2S)-1-[(2S,3S)-3-hexyl-4-oxooxetan-2-yl]tridecan-2-yl (2S)-2-formamido-4-methylpentanoate (Orlistat), 2-(hexadecyloxy)-6-methyl-4H-3,1-benzoxazin-4-one (Cetilistat), (1S,3aS,3bR,9bS,11aS)-7-(heptanoyloxy)-11a-methyl-1H,2H,3H,3aH,3bH,4H,5H,9bH,10H,11H,11aH-cyclopenta[a]phenanthren-1-yl heptanoate (Estradiol dienanthate), Hexadecyltrimethylazanium (2S)-2-(6-methoxynaphthalen-2-yl)propanoate (Cetyltrimethylammonium naproxenate), Hexadecyltrimethylazanium
(Cetrimonium), Diethyl(methyl)(2-{4-[2-(octyloxy)benzamido]benzoyloxy}ethyl)azanium (Otilonium), Pentadecyl octanoate (Hexadecyl Octanoate), Dodecyldimethyl(2-phenoxyethyl)azanium (Domiphen), Tert-butyl (2S)-2-[(2S)-4-(benzyloxy)-2-{[(tert-butoxy)carbonyl]amino}-4-oxobutanamido]-6-{[(benzyloxy)carbonyl]amino}hexanoate (Reversin 121), Bis(decyl)dimethylazanium (Didecyldimethylammonium), 2-{4-[3-(2-chloro-10H-phenothiazin-10-yl)propyl]piperazin-1-yl}ethyl heptanoate (Perphenazine enanthate), N,N,N-trimethyl-3-{[methyl({10-[methyl({[3-(trimethylazaniumyl)phenoxy]carbonyl})amino]decyl})carbamoyl]oxy}anilinium (Demecarium), N-[2-(4-{3-[(propan-2-yl)amino]pyridin-2-yl}piperazine-1-carbonyl)-1H-indol-5-yl]methanesulfonamide (Delavirdine), S-{2-[(2-chloro-4-sulfamoylphenyl)amino]-2-oxoethyl} 6-methyl-3,4-dihydroquinoline-1(2H)-carbothioate, 2,6,10,15,19,23-hexamethyltetracosane (Squalane), and N-{3-[(2-{[4-(4-methylpiperazin-1-yl)phenyl]amino}thieno[3,2-d]pyrimidin-4-yl)oxy]phenyl}prop-2-enamide (Olmutinib).

9. The compound for use according to any one of claims 1 to 8, wherein the Malaria infection is an uncomplicated, a severe complicated, a cerebral, and/or a multi-drug resistant malaria infection, such as a chloroquine resistant malaria infection, a mefloquine resistant malaria infection, a mapaerine resistant malaria infection, a quinine resistant malaria infection, an amidoquine resistant malaria infection, and/or a halofantrine resistant malaria infection, optionally wherein the Malaria infection is Malaria tropica, Malaria tertiana, and/or Malaria quartana.

10. The compound for use according to any one of claims 1 to 9, wherein the method comprises the administration of the compound to a subject, optionally wherein the administration is by oral, interactal, intramuscular, subcutaneous, intradermal, intraperitoneal, intravenous, and/or intra-pleural route, further optionally wherein the subject is a human, a monkey, a mouse, a rat, a rabbit, a reptile, a bird, a bat, a lizard, or an antelope, preferably wherein the subject is a patient, such as a comatose patient.

11. A method for identifying and/or characterizing a compound suitable for the prevention and/or treatment of a disease, the method comprising the steps of:
(a) Providing at least one parasite, such as a Plasmodium or a related species of the Apicomplexa, Amoebozoa or Euglenozoa group, and a candidate compound, wherein the parasite comprises at least one CRE Snf2L;
(b) Bringing into contact the at least one parasite and the candidate compound, and
(c) Detecting and/or quantifying the level of activity of the at least one CRE Snf2L, optionally wherein the detecting and/or quantifying the level of activity of the at least one CRE Snf2L comprises measuring the reduction of an ATPase activity of the at least one CRE Snf2L, further optionally wherein the detecting and/or quantifying the level of activity of the at least one CRE Snf2L does not comprise monitoring the basal ATPase activity of the at least one CRE Snf2L;
wherein a reduction and/or inhibition of the level of activity of the at least one CRE Snf2L of the at least one parasite contacted with the candidate compound compared to the activity of the at least one CRE Snf2L of the at least one parasite not contacted with the candidate compound indicates the candidate compound as suitable for the prevention and/or treatment of the disease.

12. A compound as identified by a method for identifying and/or characterizing a compound suitable for the prevention and/or treatment of a disease according to claim 11.

13. A pharmaceutical composition comprising the compound for use according to any one of claims 1 to 10, or a compound according to claim 12, and a pharmaceutical acceptable carrier, stabilizer and/or excipient.

14. A pharmaceutical combination comprising:
(i) the compound for use according to any one of claims 1 to 10, a compound according to claim 12, or the pharmaceutical composition according to claim 13, and
(ii) an additional anti-malarial compound, optionally wherein the additional anti-malarial compound is selected from Chloroquine, Mefloquine, Mapaerine, Quinine, Amidoquine, Tafenoquine, Pyrimethamine, Lumefantrine, Hydroxychloroquine, Sulfisoxazole, Artenimol, Clotrimazole, Diethyltoluamide, Clindamycin, Lumefantrine co-artemether, Artesunate, Sulfametopyrazine, Dapsone, Quinidine, Demeclocycline, Hydralazine, Azithromycin, Sulfadoxine, Artemotil, Primaquine, Artemether, Proguanil, Doxycycline, Ciprofloxacin, Methylene blue, Acetarsol, Acetyl sulfisoxazole, Midazolam, UCB7362, PEV3A, Cipargamin, Quercetin, Artefenomel, SJ-733, Pyronaridine, ACT-451840, Artemisinin, p-Quaterphenyl, Temefos, DSM-265, Chlorproguanil, AQ-13, Transfluthrin, Artemisone, 16-Bromoepiandrosterone, Fenpyroximate, Pafuramidine, Ganaplacide, Voacamine, QS-21, JPC-3210, and Halofantrine.

15. A kit comprising the compound for use according to any one of claims 1 to 10, the compound according to claim 12, the pharmaceutical composition according to claim 13, or the pharmaceutical combination according to claim 14, and technical instructions providing information on administration and/or dosage of the compound or the pharmaceutical composition.
